(19) Europäisches Patentamt
European Patent Office
Office européen des brevets

(11) EP 3 094 307 B1

(12) **EUROPEAN PATENT SPECIFICATION**

(45) Date of publication and mention of the grant of the patent:
**02.01.2019 Bulletin 2019/01**

(21) Application number: **14803159.4**

(22) Date of filing: **28.11.2014**

(51) Int Cl.:
***A61K 8/37*** (2006.01) ***A61K 8/42*** (2006.01)
***A61Q 3/02*** (2006.01) ***A61K 8/49*** (2006.01)

(86) International application number:
**PCT/EP2014/075924**

(87) International publication number:
**WO 2015/082339 (11.06.2015 Gazette 2015/23)**

(54) **PHOTO-CROSSLINKABLE COMPOSITIONS AS KIT FOR NAIL COATING AND APPLICATION METHODS**

LICHTVERNETZBARE ZUSAMMENSETZUNGEN ALS KIT FÜR NAGELBESCHICHTUNG UND ANWENDUNGSVERFAHREN

COMPOSITIONS PHOTORÉTICULABLES COMME KIT POUR REVÊTEMENT À ONGLES ET PROCÉDÉS D'APPLICATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.12.2013 FR 1362089**

(43) Date of publication of application:
**23.11.2016 Bulletin 2016/47**

(73) Proprietor: **L'OREAL**
**75008 Paris (FR)**

(72) Inventors:
- **KERGOSIEN, Guillaume**
  **F-92370 Chaville (FR)**
- **RIACHI, Carl**
  **F-75013 Paris (FR)**
- **LE PAPE, Marina**
  **94152 Chevilly La Rue (FR)**

(74) Representative: **Miszputen, Laurent**
**L'Oréal**
**Service DIPI**
**9 Rue Pierre Dreyfus**
**92110 Clichy (FR)**

(56) References cited:
**DE-A1-102011 102 661**
**US-A1- 2011 082 228**
**US-A1- 2011 274 633**
**US-A1- 2013 263 875**

- **Aniruddhans: "ESSTECH, Inc. - Product Catalog", , 7 June 2012 (2012-06-07), XP055076110, Retrieved from the Internet: URL:www.cenveomobile.com/i/69066/47 [retrieved on 2013-08-22]**
- **Esstech ET AL: "UV NAIL GEL PRODUCTS", , 10 May 2012 (2012-05-10), XP055075254, Retrieved from the Internet: URL:http://www.esstechinc.com/pdf/Esstech Nail Gel RM 020.pdf [retrieved on 2013-08-14]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 094 307 B1

## Description

The subject of the present invention is novel photo-crosslinkable varnish compositions. Compositions of this type preferably correspond to a base coating applied directly in contact with the nail and/or the false nail. This base coating can also be referred to as first coating in the case of a structure using a plurality of coatings of distinct compositions. This coating can thus be coated with at least one second coating. In particular, this second coating can be a top coating or a coloured coating. More particularly, the first coating can be coated with a coloured coating as second coating, which is itself coated with a top coating as third coating. The subject of the present invention is also methods for applying such compositions to nails and/or false nails, and also the use of said compositions for making up and/or caring for the nails and/or false nails.

The nail varnish compositions can be employed as a varnish base or base coat, as a product for making up the nails, or as a finishing composition, also known as a top coat, to be applied to the product for making up the nails, or else as product for the cosmetic care of the nails. These compositions can be applied both to natural nails and to false nails.

In the nail varnish field, liquid cosmetic compositions are known which are used by first depositing a coating on the nail, and then by subjecting said coating to the action of a light radiation, which causes *in situ* polymerization and/or crosslinking reactions within said coating, resulting in polymeric networks which are usually crosslinked. Such photo-crosslinkable compositions, commonly known as "UV gels", and generally based on crosslinkable compounds of (meth)acrylate monomer type, make it possible to obtain a good wear property of the coating deposited on the nail, and are described, for example, in CA 1 306 954, US 5 456 905, US 7 375 144 and FR 2 823 105.

However, conventional "soak-off" UV gels generally exhibit wear property problems when they are not applied by expert manicurists. They also generally require a step of roughening the nail aimed at sanding down the nail in order to promote the wear property of the photo-crosslinked composition in film form, which can thus considerably damage the nail. Moreover, the removal of such compositions often proves to be difficult and can require a step of scraping the nail with a metal tool, an electric sander, or an abrasive file, harmful to the integrity of the nail.

Among the patent prior art aimed at overcoming these problems, mention may be made of documents US2011/0081306, US2011/0082228, US2011/0274633 and US2012/0083547.

DE 102011102661 describes crosslinkable compositions for coating nails having good adhesion comprising at least one monomer having an unsaturated ethylenic group, at least one crosslinker having at least two ethylenic groups, at least one photoinitiator and at least one disaccharide ester.

The present invention differs from this prior art through the development of a composition which has a wear property on the nails that is better than the competing products without roughening by sanding the nail, or with only slight roughening by sanding the nail, prior to the application of the photo-crosslinkable composition.

Furthermore, some products can exhibit performance problems regarding in particular the quality of the makeup result.

Moreover, the step of removing the prior art compositions conventionally uses tools intended to scrape the surface of the nail so as to remove the photo-crosslinked film of composition previously applied, which are capable of damaging the nails.

Finally, the present invention aims to provide novel photo-crosslinkable compositions which have, after photo-crosslinking of the film, a low content of extractable compounds comprising reactive (meth)acrylate functions.

The present invention thus aims to provide novel photo-crosslinkable compositions which do not exhibit at least one of the drawbacks of the compositions mentioned above.

In particular, the present invention aims to provide photo-crosslinkable compositions which can be removed with conventional organic solvents, such as acetone, without requiring a tool which is abrasive for the nails.

In particular, the present invention aims to provide photo-crosslinkable compositions which exhibit a good compromise between wear property and makeup removal compared with the photo-crosslinkable compositions described in the prior art or which exist.

The present invention also aims to provide photo-crosslinkable compositions which allow a quality nail makeup result, in particular in terms of homogeneity of result and, where appropriate, of colour.

The present invention aims to provide photo-crosslinkable compositions which are easy to use, including by the user herself, thus making it possible to save time and money.

The present invention relates to a kit for coating a nail or false nail, and more particularly for making up a nail or false nail, comprising:

- a first photo-crosslinkable composition comprising, in a physiologically acceptable medium, at least one photo-crosslinkable compound a) comprising at least two (ALK)acrylate functions and at least one carboxylic acid function,
- a second photo-crosslinkable composition comprising, in a physiologically acceptable medium:

- at least one photo-crosslinkable compound b) comprising at least one (poly)urethane poly(ALK)acrylate compound, preferably polyurethane di(meth)acrylate compound, more preferentially polyurethane dimethacrylate compound, the photo-crosslinkable compound(s) b) comprising a (poly)oxyalkylene unit, in particular comprising a (poly)oxyethylene unit, preferably comprising from 1 to 100 oxyalkylene units, preferably from 5 to 50 oxyalkylene units, and preferentially approximately 8 to 10 oxyalkylene units, preferably identical to that contained in the first composition,
- at least one photo-crosslinkable compound c) comprising at least two carbamate units obtained by reaction with at least one diisocyanate of isophorone diisocyanate type,
- at least one (ALK)acrylate monomer, preferably (meth)acrylate monomer, more preferentially tetrahydrofurfuryl methacrylate
  in accordance with claim 1.

According to preferred embodiments corresponding to at least one of the abovementioned problems:

- the photo-crosslinkable compound(s) a) correspond(s) to formula (I) below:

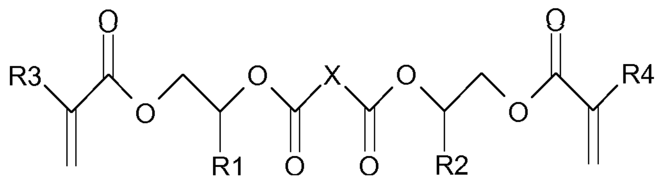

(I)

in which formula (I):

- R1 and R2, which may be identical or different, represent a hydrogen atom, or a methyl group, or a group of formula (II) below:

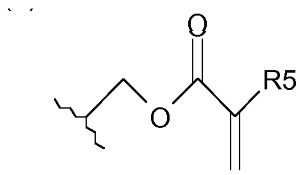

(II)

R5 representing a hydrogen atom or a methyl group,
R1 and R2 preferably representing a hydrogen atom;

- R3 and R4, which may be identical or different, represent a hydrogen atom or a methyl group;
- X represents a radical corresponding to one of formula (III), (IV), (V), (VI), (VII), (VIII), (IX) or (X) below:

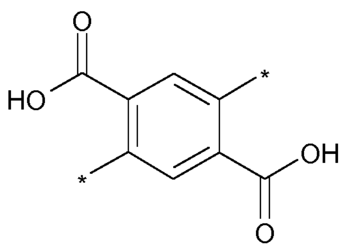

(III)

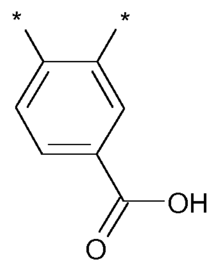

(IV)

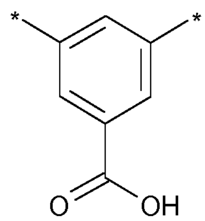

(V)

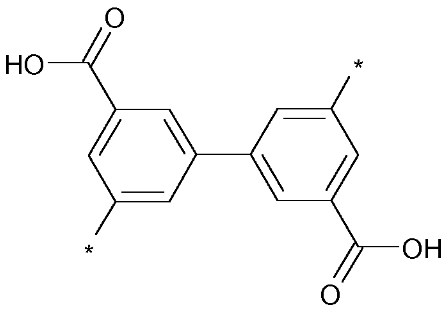

(VI)

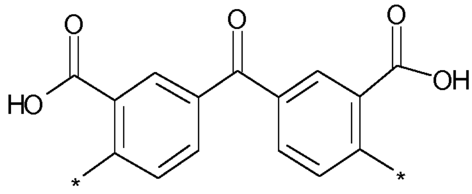

(VII)

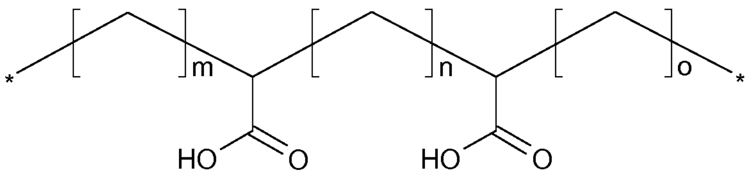

(VIII)

in which formula (VIII) m, n and o, which may be identical or different, represent an integer between 0 and 10;

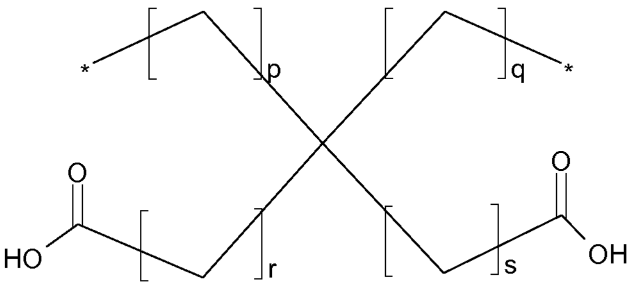

(IX)

in which formula (IX) p, q, r and s, which may be identical or different, represent an integer between 0 and 10;

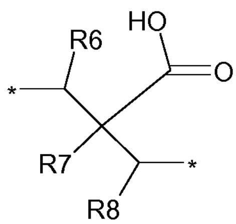

(X)

in which formula (X) R6, R7 and R8, which may be identical or different, represent a hydrogen atom or a hydroxyl group;

- the photo-crosslinkable compound(s) a) correspond(s) to formula (1-1) below:

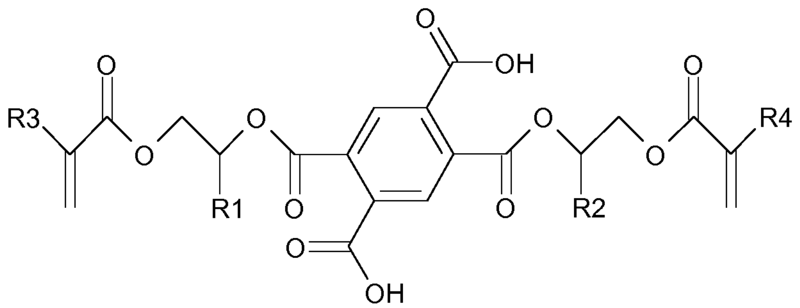

(I-1)

in which formula (I-1) R1, R2, R3 and R4 are as defined in Claim 2;

- R3 and R4 of formula (I-1) are methyl groups;
- R1 and R2 of formula (I-1), which may be identical or different, represent a hydrogen atom, a methyl group or a group of formula (II-1) below:

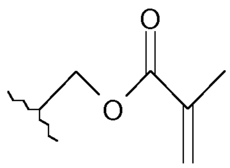

(II-1) ;

R1 and R2 preferably represent a hydrogen atom;

- the compound(s) a), corresponding to formula (I), is (are) present in the first composition in a content greater than or equal to 10% by weight, relative to the weight of the total solids of the first composition, in particular ranging from 10% to 25% by weight, preferably from 15% to 20% by weight, relative to the weight of the total solids of the first composition;
- the first composition comprises at least one photo-crosslinkable compound b) comprising at least one (poly)urethane poly(ALK)acrylate compound, preferably polyurethane di(meth)acrylate compound, more preferentially polyurethane dimethacrylate compound, the photo-crosslinkable compound(s) b) comprising a (poly)oxyalkylene unit, in particular comprising a (poly)oxyethylene unit, preferably comprising from 1 to 100 oxyalkylene units, preferably from 5 to 50 oxyalkylene units, and preferentially approximately 8 to 10 oxyalkylene units, preferably identical to that contained in the first composition;
- the compound(s) b) correspond(s) to formula (XI) below:

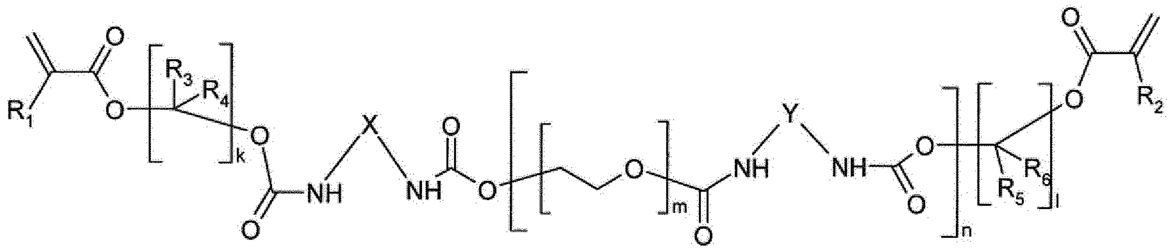

Formula (XI)

in which formula (XI):

- $R_1$, $R_2$, $R_3$, $R_4$, $R_5$ and $R_6$, which may be identical or different, represent a hydrogen atom or a $C_1$-$C_6$ alkyl chain, preferably a hydrogen atom or a methyl group,
- k and l, which may be identical or different, are between 1 and 10, preferably equal to 2,
- m is between 1 and 100, preferably between 5 and 50, preferably between approximately 8 and 10,
- n is between 1 and 10, preferably equal to 1,
- X and Y, which may be identical or different, represent a $C_1$-$C_{20}$ alkyl or cycloalkyl group;

- the compound(s) b) present in the first composition, in particular corresponding to formula (XI), is (are) present in a content greater than or equal to 20% by weight, relative to the total weight of the solids of the first composition,

in particular ranging from 25% to 50% by weight, preferably from 30% to 50% by weight, relative to the weight of the total solids of the first composition;

- the compound(s) b) present in the second composition, in particular corresponding to formula (XI), is (are) present in a content greater than or equal to 10% by weight, relative to the total weight of the solids of the second composition, in particular ranging from 25% to 80% by weight, preferably from 50% to 70% by weight, relative to the weight of the total solids of the second composition;
- the compound(s) c) correspond(s) to formula (XIII) below:

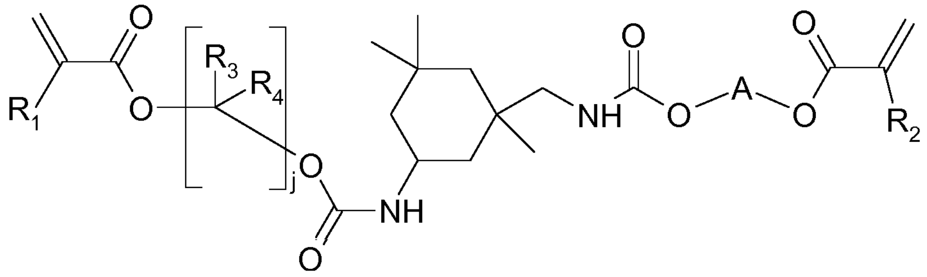

Formula (XIII)

in which formula (XIII):

- $R_1$, $R_2$, $R_3$ and $R_4$, which may be identical or different, represent a hydrogen atom or a $C_1$-$C_{10}$ alkyl chain, preferably a hydrogen atom or a methyl group,
- j is between 1 and 10, preferably equal to 2,

A representing a $C_1$-$C_{10}$ alkyl group, or a polyurethane comprising from 2 to 20 carbamate units;

- the compound(s) c) corresponding to formula (XIII), is (are) present in a content greater than or equal to 5% by weight, relative to the total weight of the solids of the second composition, in particular ranging from 10% to 80% by weight, preferably from 15% to 70% by weight, relative to the weight of the total solids of the second composition;
- the first composition and the second composition comprise at least one film-forming polymer, preferably chosen from the group consisting of poly(meth)acrylates, polysaccharides and derivatives, and of a mixture thereof, preferably a mixture thereof;
- the first composition comprises at least one film-forming polymer chosen from the group consisting of poly(meth)acrylates, and preferably a mixture of poly(meth)acrylate(s) and of polysaccharide(s) and derivatives;
- the second composition comprises at least one film-forming polymer chosen from the group of polysaccharides and derivatives;
- the film-forming polymer(s) comprise(s) at least one poly(meth)acrylate corresponding to formula (XII) below:

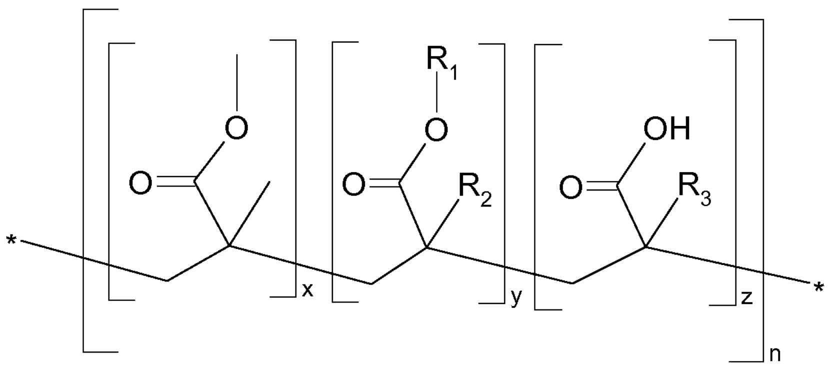

Formula (XII)

in which formula (XII):

- $R_1$, $R_2$ and $R_3$, which may be identical or different, represent a hydrogen atom or a $C_1$-$C_{10}$ alkyl group, $R_1$

preferably representing a $C_4$-$C_{10}$ alkyl group, and $R_2$ and $R_3$ preferably representing a hydrogen atom or a methyl group,

- x and y, which may be identical or different, represent an integer between 1 and 100,
- z represents an integer between 0 and 100,
- n represents an integer between 1 and 1000;

- the polysaccharide(s) and polysaccharide derivative(s) is (are) chosen from nitrocellulose and ethers and esters of polysaccharides, in particular of $C_2$-$C_4$, in particular from cellulose acetobutyrates, cellulose acetopropionates, ethylcelluloses, ethyl guars, and mixtures thereof, more preferentially chosen from nitrocellulose;
- the film-forming polymer(s) is (are) present in the first composition in a total content greater than or equal to 20% by weight, relative to the total weight of the solids of the first composition, preferably from 25% to 40% by weight, relative to the weight of the total solids of the first composition;
- the film-forming polymer(s) is (are) present in the second composition in a total content greater than or equal to 0.05% by weight, relative to the total weight of the solids of the second composition, in particular ranging from 0.1% to 10% by weight, preferably from 0.2% to 5% by weight, relative to the weight of the total solids of the second composition;
- the first composition comprises at least one volatile solvent, preferably at least one polar volatile solvent advantageously chosen from the group consisting of $C_3$-$C_6$ esters and ketones and mixtures thereof;
- the first composition has a total content of volatile solvent(s) greater than or equal to 30% by weight, relative to the total weight of the composition, in particular ranging from 50% to 70%, relative to the total weight of the first composition;
- the first composition and the second composition comprise at least one photoinitiator, the photoinitiator preferably being chosen from the group consisting of $\alpha$-hydroxy ketones, $\alpha$-amino ketones, aromatic ketones preferably combined with a hydrogen-donating compound, aromatic $\alpha$-diketones and acylphosphine oxides, and mixtures thereof, advantageously from the group consisting of acylphosphine oxides;
- the first composition comprises at least one (ALK)acrylate monomer, preferably distinct from the compounds a), b), c), preferably (meth)acrylate monomer, distinct from the compounds a), b), c), more preferentially tetrahydrofurfuryl methacrylate;
- the (ALK)acrylate monomer(s) is (are) present in the first composition in a total content greater than or equal to 0.1% by weight, relative to the total weight of the solids of the first composition, in particular ranging from 0.2% to 10% by weight, preferably from 0.5% to 5% by weight, relative to the weight of the total solids of the first composition;
- the (ALK)acrylate monomer(s) is (are) present in the second composition in a total content greater than or equal to 2% by weight, relative to the total weight of the solids of the second composition, in particular ranging from 5% to 40% by weight, preferably from 10% to 30% by weight, relative to the weight of the total solids of the second composition;
- the first composition is transparent;
- the second composition is transparent or coloured.

The present invention also relates, according to a second aspect of the invention, to a method for coating the nails and/or false nails, in particular for making up and/or caring for the nails and/or false nails, comprising at least the following steps:

A) application, to a nail or a false nail, of a first photo-crosslinkable composition of the kit as previously defined, via which a coating consisting of at least one coat of said first photo-crosslinkable composition is deposited,
B) exposure of the coated nail or false nail obtained at the end of step A) to a UV or visible light radiation,
C) application, to the first coating resulting from step A) and B), of a second composition of the kit as previously defined, via which a second coating consisting of at least one coat of said second composition is deposited,
D) exposure of the coated nail or false nail obtained at the end of step C) to a UV or visible light radiation.

The present invention relates more particularly to a method for coating the nails and/or false nails, in particular for making up and/or caring for the nails and/or false nails, comprising at least the following steps:

A) application, to a nail or a false nail, of a first photo-crosslinkable composition of the kit as previously defined, via which a coating consisting of at least one coat of said first photo-crosslinkable composition is deposited,
B) exposure of the coated nail or false nail obtained at the end of step A) to a UV or visible light radiation,
C) application, to the first coating resulting from step A) and B), of a second composition of the kit as previously defined, via which a second coating consisting of at least one coat of said second composition is deposited,
D) exposure of the coated nail or false nail obtained at the end of step C) to a UV or visible light radiation,

E) application, to the second coating resulting from step C) and D), of a third composition, distinct from the first composition and from the second composition, via which a third coating consisting of at least one coat of said third composition is deposited,

F) exposure of the coated nail or false nail obtained at the end of step E) to a UV or visible light radiation.

In such a method, the second coating is preferably a top coat, optionally free of colouring agent.

Advantageously, when a third coating of a third composition is applied, the second composition applied as second coating comprises at least one colouring agent.

Advantageously, steps C) and D) are repeated in total twice, the second composition being applied a first time and exposed to a UV or visible light radiation, then being applied a second time and exposed to a UV or visible light radiation, steps A) and B), like E) and F), preferably being respectively carried out only once.

In such methods, the second coating is preferably a or preferentially a plurality of coloured coat(s), comprising at least one colouring agent, and the third coating is preferably a top coat free of colouring agent.

***KIT***

The present invention relates to a kit comprising at least one first composition and one second composition. The first composition and the second composition are distinct from one another, having at least one difference in the chemical nature of the ingredients used or at least one difference in the content of the ingredients used, preferably both, and more preferentially having a plurality of differences in the chemical nature of the ingredients used and in the content of the ingredients used.

For the purposes of the present invention, a kit means, for example, that the first and second compositions are sold in the same packaging, or in two separate packagings in the same wrapper, or in two separate packagings in their respective wrapper with an indication for combined use of the first composition and of the second composition.

***FIRST COMPOSITION***

A kit in accordance with the present invention comprises at least one first photo-crosslinkable composition. The contents of the ingredients present in this first composition will be expressly expressed as percentage by weight, relative to the total solids of this first composition, or as percentage by weight, relative to the total weight of this first composition.

***Solids***

The first composition according to the invention advantageously comprises a solids content greater than or equal to 30%, in particular greater than or equal to 40%, and advantageously less than or equal to 60%, in particular less than or equal to 50%.

For the purposes of the present invention, the "*solids content*" denotes the content of non-volatile matter.

The solids content (abbreviated as SC) of a composition according to the invention is measured using a "Halogen Moisture Analyzer HR 73" commercial halogen desiccator from Mettler Toledo. The measurement is performed on the basis of the weight loss of a sample dried by halogen heating, and thus represents the percentage of residual matter once the volatile matter has evaporated off.

The measurement protocol is as follows:

About 2 g of the composition, hereinafter the sample, are spread on a metal crucible. The sample is photo-crosslinked under a nitrogen stream (in order to prevent the atmospheric oxygen from inhibiting the crosslinking at the surface of the sample). The metal crucible is then placed in the halogen desiccator mentioned above. The sample is then subjected to a temperature of 105°C until a constant weight is obtained. The wet mass of the sample, corresponding to its initial mass before crosslinking, and the dry mass of the sample, corresponding to its mass after crosslinking and halogen heating, are measured using a precision balance.

The experimental error associated with the measurement is of the order of plus or minus 2%.

The solids content is calculated in the following manner:

Solids content (expressed as weight percentage) = 100 × (dry mass/wet mass).

***Physiologically acceptable medium***

The cosmetic compositions according to the invention comprise a physiologically acceptable medium.

The term "physiologically acceptable medium" is intended to denote a medium that is particularly suitable for applying a composition of the invention to keratin materials.
The physiologically acceptable medium is generally adapted to the nature of the support onto which the composition has to be applied, and also to the appearance under which the composition has to be packaged.

***Photo-crosslinkable compounds***

A composition in accordance with the present invention comprises at least the photo-crosslinkable compound a), and advantageously at least the photo-crosslinkable compound a) and the photo-crosslinkable compound b).
In the context of the present invention, the term "photo-crosslinkable compounds" denotes organic compounds capable of crosslinking under the action of a light radiation, resulting in a crosslinked polymeric network.
The photo-crosslinkable compounds preferably comprise at least one (ALK)acrylate function, namely at least one $H_2C=C(R)-C(O)-O-$ function, with R preferably equal to H or ALK, it being understood that ALK represents a $C_1-C_6$, preferably $C_1-C_2$, more preferably $C_1$, alkyl group, such as $CH_3$, R preferably being equal to $CH_3$.

***Photo-crosslinkable compound(s) a)***

A first composition according to the invention comprises at least one photo-crosslinkable compound a). It can comprise a single photo-crosslinkable compound a) or a mixture of several photo-crosslinkable compounds a), preferably a single photo-crosslinkable compound a).
According to one embodiment, the compositions of the invention comprise a single photo-crosslinkable compound as defined hereinafter.
In the context of the present invention, the term "photo-crosslinkable compound" denotes an organic compound capable of crosslinking under the action of a light radiation, resulting in a crosslinked polymeric network.
The compounds a) are defined by the presence of at least one carboxylic acid function, namely a -COOH function, and at least two (ALK)acrylate functions, (ALK) acrylate functions of formula $H_2C=C(R)-C(O)-O-$, with R preferably equal to H or ALK, it being understood that ALK represents a $C_1-C_6$, preferably $C_1-C_2$, more preferably $C_1$, alkyl group, such as $CH_3$, R preferably being equal to $CH_3$.
According to one embodiment, the compounds a) comprise at least two carboxylic acid functions and at least two (meth)acrylate, preferably methacrylate, functions.
According to one embodiment, the compounds a) comprise two carboxylic acid functions and four (meth)acrylate functions, preferably four methacrylate functions.
According to one embodiment, the photo-crosslinkable compound(s) a) correspond(s) to formula (I) below:

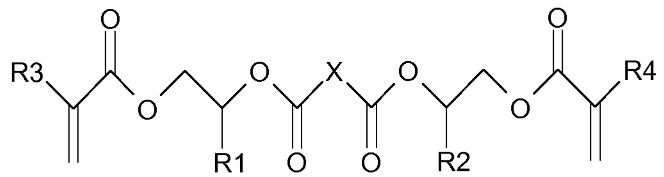

(I)

in which formula (I):

- R1 and R2, which may be identical or different, represent a hydrogen atom, or a methyl group, or a group of formula (II) below:

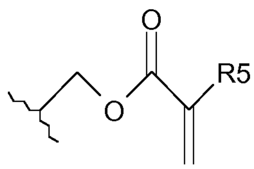

(II)

- R5 representing a hydrogen atom or a methyl group,
- R1 and R2 preferably representing a hydrogen atom;
- R3 and R4, which may be identical or different, represent a hydrogen atom or a methyl group;
- X represents a radical corresponding to one of formula (III), (IV), (V), (VI), (VII), (VIII), (IX) or (X) below:

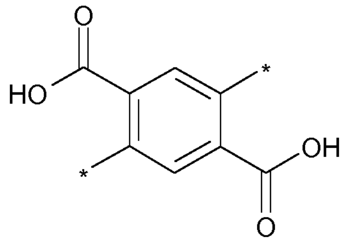

(III)

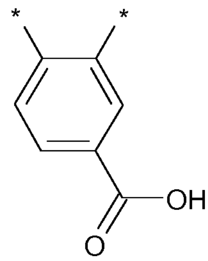

(IV)

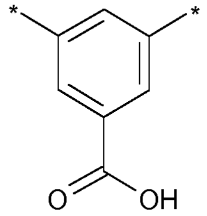

(V)

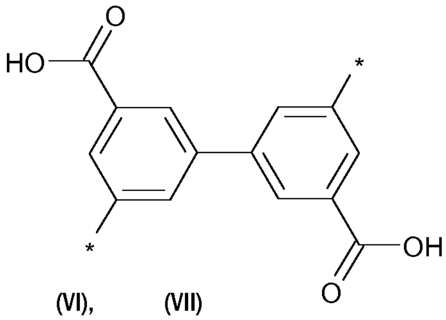

**(VI),** **(VII)**

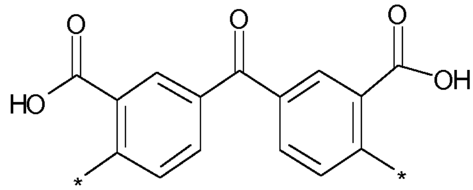

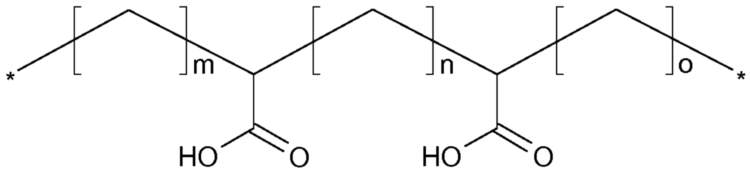

(VIII)

in which formula (VIII) m, n and o, which may be identical or different, represent an integer between 0 and 10, preferably between 0 and 1;

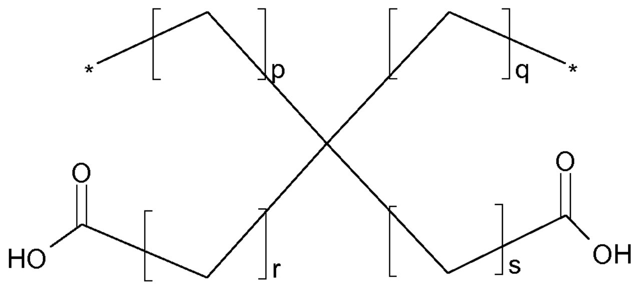

(IX)

in which formula (IX) p, q, r and s, which may be identical or different, represent an integer between 0 and 10, preferably between 0 and 1;

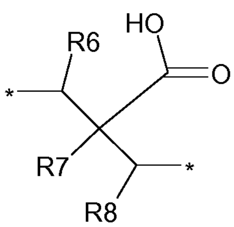

(X)

in which formula (X) R6, R7 and R8, which may be identical or different, represent a hydrogen atom or a hydroxyl group.

In the formulae above, the symbols * denote the points of attachment of the -X- radicals.

According to one embodiment, the photo-crosslinkable compound(s) a) correspond(s) to formula (I) as defined above, in which the X radical is an aromatic radical, in particular an arylene radical, and preferably a phenylene radical.

Preferably, the photo-crosslinkable compound(s) a) correspond(s) to formula (1-1) below:

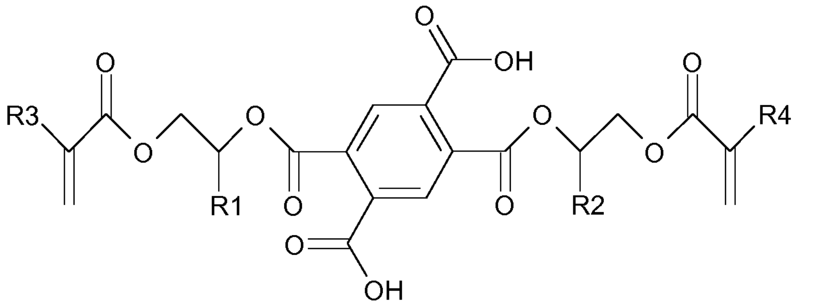

(I-1)

R1, R2, R3 and R4 being as defined above in formula (I).

According to one embodiment, the compounds a) correspond to formula (I) or (I-1) as defined above, in which R3 and R4 are methyl groups.

According to one embodiment, the compounds a) correspond to formula (I) or (I-1) as defined above, in which R1 and R2, which may be identical or different, represent a hydrogen atom, a methyl group or a group of formula (II-1) below:

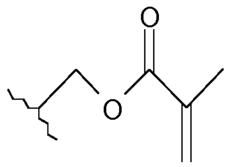

(II-1)

R1 and R2 preferably represent a hydrogen atom.

Preferentially, the photo-crosslinkable compound(s) a) correspond(s) to formula (1-2) below:

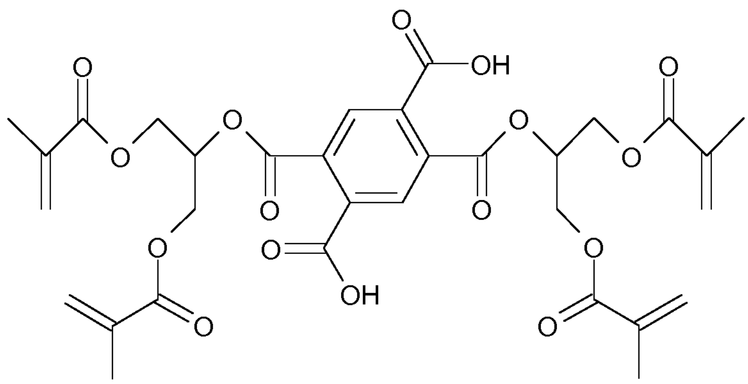

(I-2)

The compound(s) a), corresponding to formula (I), more preferably to formula (1-2), is (are) present in the first composition in a content greater than or equal to 10% by weight, relative to the weight of the total solids of the first composition, in particular ranging from 10% to 25% by weight, preferably from 15% to 20% by weight, relative to the weight of the total solids of the first composition.

***Photo-crosslinkable compound(s) b)***

A first composition of the kit according to the invention advantageously comprises at least one photo-crosslinkable compound b).

It can comprise a single photo-crosslinkable compound b) or a mixture of several photo-crosslinkable compounds b), preferably a single photo-crosslinkable compound b).

The photo-crosslinkable compound(s) b) comprise at least one (poly)urethane poly(ALK)acrylate compound comprising a (poly)oxyalkylene unit.

Generally, the term "(poly)urethane poly(ALK)acrylate compound" is intended to mean any compound comprising at least one urethane function -O-C(O)-NH-, and comprising several (ALK)acrylate functions of formula $H_2C=C(R)-C(O)-O-$, with R preferably equal to H or ALK, it being understood that ALK represents a $C_1$-$C_6$, preferably $C_1$-$C_2$, more preferably $C_1$, alkyl group, such as $CH_3$, R preferably being equal to $CH_3$.

The "urethane" function is also referred to as "carbamate" function. Preferably, the photo-crosslinkable compound(s) b) comprise several urethane or carbamate functions.

As a (poly)urethane poly(ALK)acrylate compound, polyurethane poly(ALK)acrylate compounds, especially polyurethane di(ALK)acrylate compounds, in particular polyurethane di(meth)acrylate compounds, more particularly polyurethane dimethacrylate compounds, are preferred.

Thus, the term "poly(meth)acrylate" denotes a compound comprising at least two methacrylate functions, or at least two acrylate functions, or else at least one methacrylate function and at least one acrylate function, preferably at least two methacrylate functions.

Advantageously, the average number of (meth)acrylate functions borne by the photo-crosslinkable (poly)urethane (meth)acrylate compound(s) intended to form, after crosslinking, a crosslinked polymeric network, is greater than or equal to 2, for example between 2 and 6, better still between 2 and 4, more preferentially is equal to 2.

Preferably, the photo-crosslinkable compound(s) b) therefore comprise(s) at least one polyurethane dimethacrylate compound comprising a plurality of urethane functions -O-C(O)-NH-, in particular at least two urethane functions, and a plurality of methacrylate functions of formula $H_2C=C(CH_3)-C(O)-O-$, in particular at least two methacrylate functions.

The term "(poly)oxyalkylene" is intended to mean a (poly)alkylene-oxy divalent group of which the alkylene group is linear or branched and contains from 1 to 6 carbon atoms, the alkylene group being optionally substituted with one or more hydroxyl group(s), preferably being unsubstituted as corresponds to $-[CH_2]_2-O-$ or- $O-[CH_2]_2-$.

The term "(poly)urethane poly(ALK)acrylate compound comprising a (poly)oxyalkylene unit" is thus intended to mean that the photo-crosslinkable compound(s) b) comprise(s) at least one (poly)($C_1$-$C_6$, preferably $C_2$)oxyalkylenated divalent group comprising from 1 to 100 alkylene-oxy units, in particular from 5 to 50 alkylene-oxy units, and more particularly from approximately 8 to 10 alkylene-oxy units. Preferably, these (poly)oxyalkylenated groups are polyoxyethylenated groups.

The photo-crosslinkable compound(s) b) preferably correspond(s) to formula (XI) below:

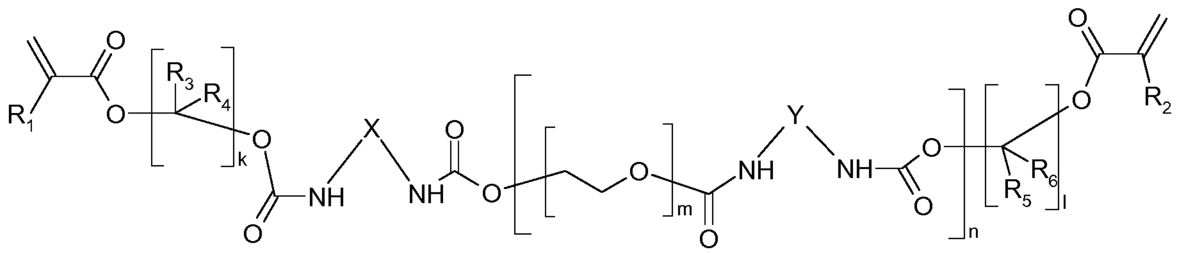

Formula (XI)

in which formula (XI):

- $R_1$, $R_2$, $R_3$, $R_4$, $R_5$ and $R_6$, which may be identical or different, represent a hydrogen atom or a $C_1$-$C_6$ alkyl chain, preferably a hydrogen atom or a methyl group,
- k and l, which may be identical or different, are between 1 and 10, preferably equal to 2,
- m is between 1 and 100, preferably between 5 and 50, preferably between approximately 8 and 10,
- n is between 1 and 10, preferably equal to 1,
- X and Y, which may be identical or different, represent a $C_1$-$C_{20}$ alkyl or cycloalkyl group.

This (these) photo-crosslinkable compound(s) b), in particular of formula (XI), advantageously has (have) a molecular weight greater than or equal to 1000 g/mol, in particular ranging from 1000 to 5000 g/mol, preferably from

1000 to 3000 g/mol.

The compound(s) b) present in the first composition, in particular corresponding to formula (XI), is (are) present in a content greater than or equal to 20% by weight, relative to the total weight of the solids of the first composition, in particular ranging from 25% to 50% by weight, preferably from 30% to 50% by weight, relative to the weight of the total solids of the first composition.

In the first composition, the photo-crosslinkable compound(s) a) of formula (I), and, when present, the photo-crosslinkable compound(s) b), in particular of formula (XI), is (are) preferably present in a respective total content such that the weight ratio of the photo-crosslinkable compound(s) a), in particular of formula (I), and of the photo-crosslinkable compound(s) b), in particular of formula (XI), ranges from 0.1 to 2, in particular from 0.25 to 1.

***(ALK)acrylate monomer(s)***

The first composition of the kit according to the invention may comprise at least one (ALK)acrylate monomer, preferably at least one (meth)acrylate monomer, such as a tetrahydrofurfuryl methacrylate compound.

ALK represents, when present in the monomer, a $C_1$-$C_6$, preferably $C_1$-$C_2$, more preferably $C_1$, alkyl group, such as $CH_3$, R preferably being equal to $CH_3$.

In particular, this (these) (ALK)acrylate monomer(s), in particular (meth)acrylate monomer(s), present in the first coat applied to the base coat contribute to improving the wear property and the mechanical properties.

The (ALK)acrylate monomer(s) may be present in the first composition in a total content greater than or equal to 0.1% by weight, relative to the total weight of the solids of the first composition, in particular ranging from 0.2% to 10% by weight, preferably from 0.5% to 5% by weight, relative to the weight of the total solids of the first composition.

***Film-forming polymer(s)***

The first composition of the kit according to the invention advantageously also comprises at least one film-forming polymer.

It can comprise a single film-forming polymer or a mixture of several film-forming polymers, preferably a mixture of several film-forming polymers.

The function of the film-forming polymer(s) is to confer a wear property on the photo-crosslinkable composition and also to promote removal of said composition.

Preferably, the first compositions according to the invention comprise at least two film-forming polymers.

The film-forming polymer(s) is (are) present in the first composition in a total content greater than or equal to 20% by weight, relative to the total weight of the solids of the first composition, preferably from 25% to 40% by weight, relative to the weight of the total solids of the first composition.

For the purposes of the present invention, the term "film-forming polymer" denotes a polymer that is capable, by itself (i.e. in the absence of an auxiliary film-forming agent or of an external stimulus for example of the UV type), of forming an isolable and in particular continuous and adherent film, on a support, in particular on the nails.

This film-forming polymer may be chosen from the group consisting of synthetic polymers, of radical type or of polycondensate type, and polymers of natural origin, and mixtures thereof.

A film-forming polymer that is suitable for the invention can be chosen from at least one poly(meth)acrylate compound, in particular from (meth)acrylate homopolymers and copolymers, preferably from (meth)acrylate copolymers.

The poly(meth)acrylate compound(s), in particular the (meth)acrylate copolymer(s), present in the composition is (are) advantageously capable of being obtained by:

i) polymerization of at least one methyl methacrylate (MMA) monomer and of at least one acrylic or methacrylic acid (AA or MAA) monomer, or

ii) polymerization of at least one methyl methacrylate (MMA) monomer, of at least one monomer with a glass transition temperature below 30°C, such as butyl methacrylate (BMA), butyl acrylate (BA) or 2-ethylhexyl acrylate (2-EHA), and optionally of at least one acrylic acid (AA) or methacrylic acid (MAA) monomer.

A first composition according to the invention preferably comprises at least one poly(meth)acrylate film-forming polymer of type ii) obtained by polymerization of at least one methyl methacrylate (MMA) monomer, of at least one monomer with a glass transition temperature below 30°C, such as butyl methacrylate (BMA), butyl acrylate (BA) or 2-ethylhexyl acrylate (2-EHA), and optionally of at least one acrylic acid (AA) or methacrylic acid (MAA) monomer.
Such a poly(meth)acrylate film-forming polymer of type ii) preferably corresponds to formula (XII) below:

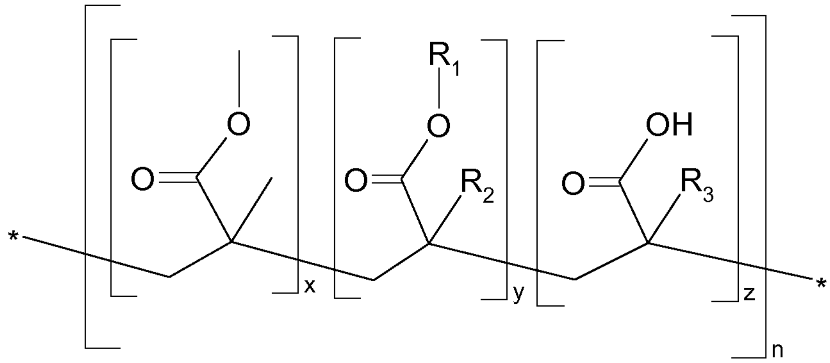

Formula (XII)

in which formula (XII):

- $R_1$, $R_2$ and $R_3$, which may be identical or different, represent a hydrogen atom or a $C_1$-$C_{10}$ alkyl group, $R_1$ preferably representing a $C_4$-$C_{10}$ alkyl group, and $R_2$ and $R_3$ preferably representing a hydrogen atom or a methyl group,
- x and y, which may be identical or different, represent an integer between 1 and 100,
- z represents an integer between 0 and 100,
- n represents an integer between 1 and 1000.

Preferably, a composition according to the invention comprises at least one film-forming polymer c) chosen from at least one polyacrylate compound of formula (XII).

As a variant or preferably additionally, a film-forming polymer that is suitable for the invention can be chosen from polysaccharides and polysaccharide derivatives, such as derivatives of cellulose or of guar gum. A preferential polysaccharide derivative suitable for the invention may be nitrocellulose or a polysaccharide ester or alkyl ether.

The term "polysaccharide ester or alkyl ether" denotes a polysaccharide made up of repeat units comprising at least two identical or different rings and having a degree of substitution per saccharide unit of between 1.9 and 3, preferably between 2.2 and 2.9 and more particularly between 2.4 and 2.8. The term "substitution" denotes the functionalization of the hydroxyl groups to give ester and/or alkyl ether functions, and/or the functionalization of the carboxylic groups to give ester functions.

In other words, it may be a polysaccharide, partially or totally substituted with ester and/or alkyl ether groups. Preferably, the hydroxyl groups may be substituted with ester and/or alkyl ether functions of $C_2$-$C_4$.

Mention may in particular be made of cellulose esters (such as cellulose acetobutyrates or cellulose acetopropionates), cellulose alkyl ethers (for instance ethylcelluloses), and ethyl guars.

A film-forming polymer that is suitable for the invention can be chosen from synthetic polymers such as polyurethanes, acrylic polymers, vinyl polymers, polyvinyl butyrals, alkyd resins and ketone/aldehyde resins, resins derived from aldehyde condensation products, such as arylsulfonamide-formaldehyde resins, for instance toluenesulfonamide-formaldehyde resin, arylsulfonamide-epoxy resins or ethyl tosylamide resins.

A film-forming polymer that is suitable for the invention can also be chosen from polymers of natural origin, such as plant resins, such as dammar resins, elemi resins, copal resins, and benzoin; gums such as shellac, sandarac gum and mastic gum.

Use may in particular be made, as film-forming polymers, of the toluenesulfonamide/formaldehyde resins Ketjentflex MS80 from the company Akzo or Santolite MHP or Santolite MS 80 from the company Faconnier or Resimpol 80 from the company Pan Americana, the alkyd resin Beckosol ODE 230-70-E from the company Dainippon, the acrylic resin Acryloid B66 from the company Rohm & Haas, the polyurethane resin Trixene PR 4127 from the company Baxenden or the acetophenone/formaldehyde resin sold under the reference Synthetic Resin SK by Degussa.

According to one particular preferred embodiment, the film-forming polymer is chosen from the group consisting of polysaccharides and polysaccharide derivatives, preferably from nitrocellulose and polysaccharide ethers and esters, in particular of $C_2$-$C_4$, and more preferentially from cellulose acetobutyrates, cellulose acetopropionates, ethylcelluloses, ethyl guars, and mixtures thereof.

According to one advantageous embodiment, the film-forming polymer is chosen from the group consisting of nitrocellulose, cellulose acetopropionate, cellulose acetobutyrate, and (meth)acrylate homopolymers and copolymers, and mixtures thereof.

According to one advantageous embodiment, the first compositions of the invention comprise at least one film-forming polymer chosen from nitrocellulose.

According to this particular embodiment, the ratio of the weight of the film-forming polymer(s) chosen from the group consisting of polysaccharides and polysaccharide derivatives, in particular the weight of nitrocellulose, to the weight of the photo-crosslinkable compounds, in particular the sum of the respective weight of the photo-crosslinkable compounds a) and b), is less than or equal to 1, and preferentially between 0.3 and 1.

According to one advantageous embodiment, the ratio of the total weight of film-forming polymer(s), in particular chosen from the group consisting of poly(meth)acrylate compound(s), in particular of formula (XII), and the polysaccharides and polysaccharide derivatives, in particular nitrocellulose, to the weight of the photo-crosslinkable compounds, in particular the sum of the respective weight of the photo-crosslinkable compounds a) and b), and optionally of one or more (meth)acrylate monomers, such as a tetrahydrofurfuryl methacrylate compound, is less than or equal to 1, and preferentially between 0.3 and 1.

***Volatile solvent(s)***

The first composition of the kit according to the invention also advantageously comprises at least one volatile solvent.

It can therefore comprise a single volatile solvent or a mixture of several volatile solvents, preferably a mixture of several volatile solvents.

The first composition has a total content of volatile solvent(s) greater than or equal to 30% by weight, relative to the total weight of the composition, in particular ranging from 50% to 70%, relative to the total weight of the composition.

For the purposes of the invention, the term "volatile solvent" is intended to mean a solvent that is capable of evaporating on contact with keratin materials in less than one hour, at ambient temperature and atmospheric pressure.

The volatile solvent(s) of the invention are solvents which are liquid at ambient temperature and which have a non-zero vapour pressure, at ambient temperature and atmospheric pressure, ranging in particular from 50 Pa to 40 000 Pa (0.375 to 300 mmHg), in particular ranging from 100 Pa to 26 664 Pa (0.75 to 200 mmHg) and more particularly ranging from 1000 Pa to 13 332 Pa (7.5 to 100 mmHg).

Such solvents aim in particular to fluidize and reduce the solids of the composition.

Preferably, the solvents are chosen from polar solvents.

For the purposes of the present invention, the term "polar" solvent is intended to mean a solvent, or an oil, of which the solubility parameter calculated above its melting point $\delta_a$ is other than 0 $(J/cm^3)^{1/2}$.

The definition and calculation of the solubility parameters in the Hansen three-dimensional solubility space are described in the article by C.M. Hansen: "The three dimensional solubility parameters" J. Paint Technol. 39, 105 (1967).

According to this Hansen space:

- $\delta_D$ characterizes the London dispersion forces derived from the formation of dipoles induced during molecular impacts;
- $\delta_p$ characterizes the Debye interaction forces between permanent dipoles and also the Keesom interaction forces between induced dipoles and permanent dipoles;
- $\delta_h$ characterizes the specific interaction forces (such as hydrogen bonding, acid/base, donor/acceptor, etc.); and
- $\delta_a$ is determined by the equation: $\delta_a = (\delta_p^2 + \delta_h^2)^{1/2}$.

The parameters $\delta_p$, $8_h$, $\delta_D$ and $\delta_a$ are expressed in $(J/cm^3)^{1/2}$.

In particular, the term "polar" solvent is intended to mean a solvent of which the chemical structure is formed essentially from, or even consists of, carbon and hydrogen atoms, and which comprises at least one highly electronegative heteroatom such as an oxygen, nitrogen, silicon or phosphorus atom.

Preferably, this polar volatile solvent is chosen from the group consisting of $C_3$-$C_6$ esters and ketones and mixtures thereof.

By way of polar volatile solvent, mention may in particular be made of acetone, methyl ethyl ketone, methyl isobutyl ketone, cyclohexanone and alkyl acetates in which the alkyl group comprises from 2 to 5 carbon atoms, such as methyl acetate, ethyl acetate, propyl acetate, n-propyl acetate, isopropyl acetate, n-butyl acetate, isobutyl acetate and tert-butyl acetate.

Preferably, the polar volatile solvent is chosen from the group consisting of ethyl acetate, propyl acetate, such as n-propyl or isopropyl acetate, n-butyl, isobutyl or tert-butyl acetate, isopropanol, and a mixture or mixtures thereof.

According to one preferred embodiment, the solvent is a mixture of butyl acetate, ethyl acetate and propyl acetate.

The butyl acetate, the ethyl acetate and the isopropanol are preferably present in the first composition in a respective content ranging respectively from 15% to 35% by weight, from 15% to 30% by weight, and from 5% to 15% by weight, relative to the total weight of the first composition.

***Photoinitiator(s)***

The first composition of the kit according to the invention also advantageously comprises at least one photoinitiator.

It can comprise a single photoinitiator or a mixture of several photoinitiators, preferably a single photoinitiator.

The photoinitiators that can be used according to the present invention are known in the art and are described, for example, in "Les photoinitiateurs dans la reticulation des revetements" ["Photoinitiators in the crosslinking of coatings"], G. Li Bassi, Double Liaison - Chimie des Peintures, no. 361, November 1985, pp. 34-41; "Applications industrielles de la polymerisation photoinduite" ["Industrial applications of photoinduced polymerization"], Henri Strub, L'Actualite Chimique, February 2000, pp. 5-13; and "Photopolymères: considerations théoriques et reaction de prise" ["Photopolymers: theoretical considerations and setting reaction"], Marc, J.M. Abadie, Double Liaison - Chimie des Peintures, no. 435-436, 1992, pp. 28-34.

These photoinitiators encompass:

- α-hydroxy ketones, sold, for example, under the names Darocur® 1173 and 4265, Irgacure® 184, 2959, and 500 by the company BASF, and Additol® CPK by the company Cytec,
- α-amino ketones, sold, for example, under the names Irgacure® 907 and 369 by the company BASF,
- aromatic ketones, sold, for example, under the name Esacure® TZT by Lamberti. Mention may also be made of the thioxanthones sold, for example, under the name Esacure® ITX by Lamberti, and quinones. These aromatic ketones usually require the presence of a hydrogen-donating compound such as tertiary amines and in particular alkanolamines. Mention may in particular be made of the tertiary amine Esacure® EDB sold by the company Lamberti;
- α-dicarbonyl derivatives, the most common representative of which is benzyl dimethyl ketal, sold under the name Irgacure® 651 by BASF. Other commercial products are sold by the company Lamberti under the name Esacure® KB1, and
- acylphosphine oxides such as, for example, the bis-acylphosphine oxides (BAPOs) sold, for example, under the names Irgacure® 819, 1700 and 1800 and Darocur® 4265, Lucirin® TPO and Lucirin® TPO-L by the company BASF. Preferably, the photoinitiator is chosen from the group consisting of α-hydroxy ketones, α-amino ketones, aromatic ketones preferably combined with a hydrogen-donating compound, aromatic α-diketones and acylphosphine oxides, and mixtures thereof.

An acylphosphine oxide is preferably used in the photo-crosslinkable composition of the invention.

By way of photoinitiator, mention may be made of Lucirin® TPO-L (BASF).

The total content of photoinitiator(s) depends on a large number of factors such as, for example, the reactivity of the various components of the mixture, the presence of a colouring agent or colouring agents, the intensity of the light source or the exposure time.

In order to obtain the desired properties, the photoinitiator(s) is (are) preferably present in the first composition in a total content greater than or equal to 0.1% by weight, relative to the total weight of the first photo-crosslinkable composition, preferably ranging from 0.2% to 5% by weight, relative to the total weight of the first photo-crosslinkable composition.

***Other constituents***

The first composition of a kit according to the invention can also contain adjuvants, or additives, chosen in particular from stabilizers, colouring agents such as pigments, plasticizers, coalescers, preservatives, thickeners, fragrances, cosmetic nail care active agents, spreading agents, antifoams and dispersants.

Needless to say, those skilled in the art will take care to choose these optional adjuvants and additives such that the advantageous properties of the composition according to the invention are not, or are virtually not, adversely affected by the envisaged addition.

When the composition comprises colouring agents, the absorption spectrum of the colouring agents used should in particular be adapted to that of the photoinitiators, or conversely the absorption spectrum of the photoinitiators to that of the colouring agents used, in order to avoid these two types of compounds absorbing light at the same wavelengths. This is because the absorption of light by the colouring agents would render almost totally ineffective the photoinitiators present beyond a certain depth of the coating.

Preferably, the first composition of the kit according to the invention is transparent.

As used herein, the term "transparent" means that the composition has a HAZEBYK index of less than 5 as measured with a KYKHAZEGLOSS brilliance meter.

According to one embodiment, the first composition of the kit according to the invention also comprises a colouring agent chosen from the group consisting of soluble dyes, pigments, nacres and glitter flakes.

The colouring agent(s) can be present in the first composition in a total content greater than or equal to 0.1% by weight, relative to the total weight of the coat, preferably ranging from 0.1% to 5%, advantageously from 0.2% to 1% by weight, relative to the total weight of the first composition.

The term "soluble dyes" should be understood as meaning organic, inorganic or organometallic compounds which are soluble in the composition of the invention and intended to colour said composition.

The dyes are, for example, Sudan red, DC red 17, DC green 6, β-carotene, soybean oil, Sudan brown, DC yellow 11, DC violet 2, DC orange 5 and quinoline yellow.

The term "pigments" should be understood as meaning white or coloured and inorganic or organic particles of any shape which are insoluble in the composition of the invention and which are intended to colour said composition.

The term "nacres" should be understood as meaning iridescent particles of any shape, in particular produced by certain molluscs in their shell, or else synthesized.

The pigments may be white or coloured, and inorganic and/or organic. Among the inorganic pigments that may be mentioned are titanium dioxide, optionally surface-treated, zirconium oxide or cerium oxide, and also zinc oxide, iron (black, yellow or red) oxide or chromium oxide, manganese violet, ultramarine blue, chromium hydrate and ferric blue, and metal powders, for instance aluminium powder and copper powder.

Among the organic pigments that may be mentioned are carbon black, pigments of D & C type and lakes based on cochineal carmine or on barium, strontium, calcium or aluminium.

Mention may also be made of effect pigments, such as particles comprising an organic or inorganic and natural or synthetic substrate, for example glass, acrylic resins, polyester, polyurethane, polyethylene terephthalate, ceramics or aluminas, which may or may not be covered with metal substances, such as aluminium, gold, copper or bronze, or with metal oxides, such as titanium dioxide, iron oxide or chromium oxide, or with inorganic or organic pigments, and mixtures thereof.

The nacreous pigments can be chosen from white nacreous pigments, such as mica covered with titanium or with bismuth oxychloride, coloured nacreous pigments, such as titanium mica covered with iron oxides, titanium mica covered with in particular ferric blue or with chromium oxide, or titanium mica covered with an organic pigment of the abovementioned type, and nacreous pigments based on bismuth oxychloride.

Use may also be made of pigments with goniochromatic properties, in particular liquid crystal or multilayer pigments.

Optical brighteners or fibres optionally coated with optical brighteners can also be used.

A first composition of the kit according to the invention can additionally comprise one or more fillers, in particular in a content ranging from 0.01% to 50% by weight, relative to the total weight of the composition, preferably ranging from 0.01% to 30% by weight.

The term "fillers" should be understood as meaning colourless or white, inorganic or synthetic particles of any shape, which are insoluble in the medium of the composition, irrespective of the temperature at which the composition is manufactured. These fillers serve in particular to modify the rheology or the texture of the composition.

The fillers may be inorganic or organic and of any shape, platelet-shaped, spherical or oblong, irrespective of the crystallographic form (for example lamellar, cubic, hexagonal, orthorhombic, etc.). Mention may be made of talc, mica, silica, kaolin, polyamide (Nylon®) powder (Orgasol® from Atochem), poly-p-alanine powder and polyethylene powder, powders of tetrafluoroethylene polymers (Teflon®), lauroyllysine, starch, boron nitride, hollow polymeric microspheres such as those of polyvinylidene chloride/acrylonitrile, for instance Expancel® (Nobel Industrie) or of acrylic acid copolymers (Polytrap® from the company Dow Corning) and silicone resin microbeads (for example Tospearls® from Toshiba), elastomeric polyorganosiloxane particles, precipitated calcium carbonate, magnesium carbonate, magnesium hydrogen carbonate, hydroxyapatite, hollow silica microspheres (Silica Beads® from Maprecos), glass or ceramic microcapsules, and metal soaps derived from organic carboxylic acids containing from 8 to 22 carbon atoms and preferably from 12 to 18 carbon atoms, for example zinc stearate, magnesium stearate or lithium stearate, zinc laurate or magnesium myristate.

***SECOND COMPOSITION***

A kit in accordance with the present invention comprises, in addition to the first photo-crosslinkable composition, at least one second composition. The contents of the ingredients present in this second composition will be expressly expressed as percentage by weight, relative to the total solids of this second composition, or as percentage by weight, relative to the total weight of this second composition.

***Solids***

The second composition according to the invention advantageously comprises a solids content greater than or equal to 30%, preferably greater than or equal to 40%, more preferably greater than or equal to 50%, for example

ranging from 40% to 80%, better still from 50% to 70%.

The definition, the material and the measuring protocol are as previously described with reference to the first composition.

***Physiologically acceptable medium***

The second composition according to the invention comprises a physiologically acceptable medium.

***Photo-crosslinkable compounds***

A second composition in accordance with the present invention comprises at least the photo-crosslinkable compounds b) and c) and at least one (ALK)acrylate monomer.

In the context of the present invention, the term "photo-crosslinkable compounds" denotes organic compounds capable of crosslinking under the action of a light radiation, resulting in a crosslinked polymeric network.

The photo-crosslinkable compounds preferably comprise at least one (ALK)acrylate function, namely at least one $H_2C=C(R)-C(O)-O-$ function. The photo-crosslinkable compound(s) a) is (are) defined by the presence of at least one carboxylic acid function, namely one -COOH function, and at least one (ALK)acrylate function, namely at least one $H_2C=C(R)-C(O)-O-$ function, with R preferably equal to H or ALK, it being understood that ALK represents a $C_1$-$C_6$, preferably $C_1$-$C_2$, more preferably $C_1$, alkyl group, such as $CH_3$, R preferably being equal to $CH_3$.

***Photo-crosslinkable compound(s) b)***

A second composition according to the invention comprises at least one photo-crosslinkable compound b).

It can comprise a single photo-crosslinkable compound b) or a mixture of several photo-crosslinkable compounds b), preferably a single photo-crosslinkable compound b).

The photo-crosslinkable compound(s) b) comprise(s) at least one (poly)urethane poly(ALK)acrylate compound comprising a (poly)oxyalkylene unit.

Generally, the term "(poly)urethane poly(ALK)acrylate compound" is intended to mean any compound comprising at least one urethane function -O-C(O)-NH-, and comprising several (ALK)acrylate functions of formula $H_2C=C(R)-C(O)-O-$, with R preferably equal to H or ALK, it being understood that ALK represents a $C_1$-$C_6$, preferably $C_1$-$C_2$, more preferably $C_1$, alkyl group, such as $CH_3$, R preferably being equal to $CH_3$.

The "urethane" function is also referred to as "carbamate" function. Preferably, the photo-crosslinkable compound(s) b) comprise several urethane or carbamate functions.

As a (poly)urethane poly(ALK)acrylate compound, polyurethane poly(ALK)acrylate compounds, especially polyurethane di(ALK)acrylate compounds, in particular polyurethane di(meth)acrylate compounds, more particularly polyurethane dimethacrylate compounds, are preferred.

Thus, the term "poly(meth)acrylate" denotes a compound comprising at least two methacrylate functions, or at least two acrylate functions, or else at least one methacrylate function and at least one acrylate function, preferably at least two methacrylate functions.

Advantageously, the average number of (meth)acrylate functions borne by the photo-crosslinkable (poly)urethane (meth)acrylate compound(s) intended to form, after crosslinking, a crosslinked polymeric network, is greater than or equal to 2, for example between 2 and 6, better still between 2 and 4, more preferentially is equal to 2.

Preferably, the photo-crosslinkable compound(s) b) therefore comprise(s) at least one polyurethane dimethacrylate compound comprising a plurality of urethane functions -O-C(O)-NH-, in particular at least two urethane functions, and a plurality of methacrylate functions of formula $H_2C=C(CH_3)-C(O)-O-$, in particular at least two methacrylate functions.

The term "(poly)oxyalkylene" is intended to mean a (poly)alkylene-oxy divalent group of which the alkylene group is linear or branched and contains from 1 to 6 carbon atoms, the alkylene group being optionally substituted with one or more hydroxyl group(s), preferably being unsubstituted as corresponds to $-[CH_2]_2-O-$ or- $O-[CH_2]_2-$.

The term "(poly)urethane poly(ALK)acrylate compound comprising a (poly)oxyalkylene unit" is thus intended to mean that the photo-crosslinkable compound(s) b) comprise(s) at least one (poly)($C_1$-$C_6$, preferably $C_2$)oxyalkylenated divalent group comprising from 1 to 100 alkylene-oxy units, in particular from 5 to 50 alkylene-oxy units, and more particularly from approximately 8 to 10 alkylene-oxy units. Preferably, these (poly)oxyalkylenated groups are polyoxyethylenated groups.

The photo-crosslinkable compound(s) b) preferably correspond(s) to formula (XI) below:

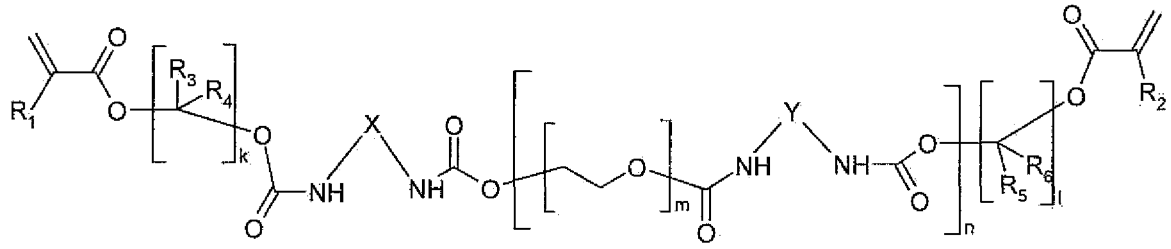

Formula (XI)

in which formula (XI):

- $R_1$, $R_2$, $R_3$, $R_4$, $R_5$ and $R_6$, which may be identical or different, represent a hydrogen atom or a $C_1$-$C_6$ alkyl chain, preferably a hydrogen atom or a methyl group,
- k and l, which may be identical or different, are between 1 and 10, preferably equal to 2,
- m is between 1 and 100, preferably between 5 and 50, preferably between approximately 8 and 10,
- n is between 1 and 10, preferably equal to 1,
- X and Y, which may be identical or different, represent a $C_1$-$C_{20}$ alkyl or cycloalkyl group.

This (these) photo-crosslinkable compound(s) b), in particular of formula (XI), advantageously has (have) a molecular weight greater than or equal to 1000 g/mol, in particular ranging from 1000 to 5000 g/mol, preferably from 1000 to 3000 g/mol.

The compound(s) b) present in the second composition, in particular corresponding to formula (XI), is (are) present in a content greater than or equal to 10% by weight, relative to the total weight of the solids of the second composition, in particular ranging from 25% to 80% by weight, preferably from 50% to 70% by weight, relative to the weight of the total solids of the second composition.

***Photo-crosslinkable compound(s) c)***

The second composition according to the invention comprises at least one photo-crosslinkable compound c) comprising at least two carbamate units obtained by reaction with at least one diisocyanate of isophorone diisocyanate type.

The compound(s) c) advantageously correspond(s) to formula (XIII) below:

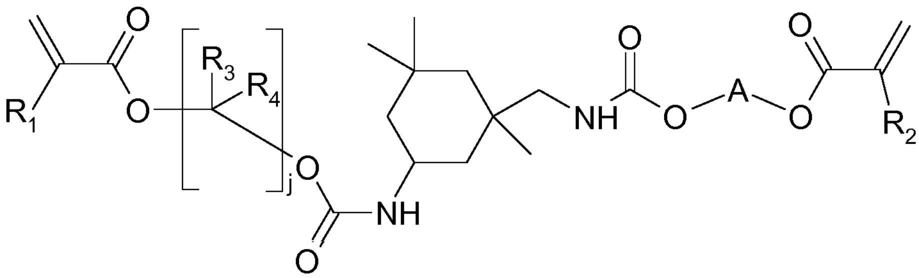

Formula (XIII)

in which formula (XIII):

- $R_1$, $R_2$, $R_3$ and $R_4$, which may be identical or different, represent a hydrogen atom or a $C_1$-$C_{10}$ alkyl chain, preferably a hydrogen atom or a methyl group,
- j is between 1 and 10, preferably equal to 2,
- A represents a $C_1$-$C_{10}$ alkyl group, or a polyurethane comprising from 2 to 20 carbamate units.

The compound(s) c), corresponding to formula (XIII), is (are) present in the second composition in a content greater than or equal to 5% by weight, relative to the total weight of the solids of the second composition, in particular ranging from 10% to 80% by weight, preferably from 15% to 70% by weight, relative to the weight of the total solids of the second composition.

In the second composition, the photo-crosslinkable compound(s) b), in particular of formula (XI), and the photo-crosslinkable compound(s) c), in particular of formula (XIII), are preferably present in a respective total content such that the weight ratio of the photo-crosslinkable compound(s) b), in particular of formula (XI), and of the photo-crosslinkable compound(s) c), in particular of formula (XIII), ranges from 0.1 to 10, in particular from 0.25 to 5.

***(ALK)acrylate monomer(s)***

The second composition according to the invention comprises at least one (ALK)acrylate monomer, preferably at least one (meth)acrylate monomer, such as a tetrahydrofurfuryl methacrylate compound.

ALK represents, when present in the monomer, a $C_1$-$C_6$, preferably $C_1$-$C_2$, more preferably $C_1$, alkyl group, such as $CH_3$, R preferably being equal to $CH_3$.

In particular, this (these) (ALK)acrylate monomer(s), in particular (meth)acrylate monomer(s), present in the second coat applied to the base coat contribute to improving the wear property and the mechanical properties.

This (these) (ALK)acrylate monomer(s) is (are) present in the second composition in a total content greater than or equal to 2% by weight, relative to the total weight of the solids of the second composition, in particular ranging from 5% to 40% by weight, preferably from 10% to 30% by weight, relative to the weight of the total solids of the second composition.

In the second composition, the photo-crosslinkable compound(s) b), in particular of formula (XI), the photo-crosslinkable compound(s) c), in particular of formula (XIII), and the (ALK)acrylate monomer(s) are preferably present in a respective total content such that the weight ratio of the (ALK)acrylate monomer(s) to the sum of the photo-crosslinkable compound(s) b), in particular of formula (XI), and of the photo-crosslinkable compound(s) c), in particular of formula (XIII), ranges from 0.1 to 2, in particular from 0.2 to 0.5.

***Film-forming polymer(s)***

The second composition according to the invention advantageously also comprises at least one film-forming polymer as previously defined.

It can comprise a single film-forming polymer or a mixture of several film-forming polymers, preferably a single film-forming polymer.

The film-forming polymer(s) is (are) present in the second composition in a total content greater than or equal to 0.05% by weight, relative to the total weight of the solids of the second composition, in particular ranging from 0.1% to 10% by weight, preferably from 0.2% to 5% by weight, relative to the weight of the total solids of the second composition;

This film-forming polymer may be chosen from the group consisting of synthetic polymers, of radical type or of polycondensate type, and polymers of natural origin, and mixtures thereof.

A film-forming polymer that is suitable for the invention can be chosen from at least one poly(meth)acrylate compound, in particular from (meth)acrylate homopolymers and copolymers, preferably from (meth)acrylate copolymers.

The poly(meth)acrylate compound(s), in particular the (meth)acrylate copolymer(s), present in the composition is (are) advantageously capable of being obtained by:

iii) polymerization of at least one methyl methacrylate (MMA) monomer and of at least one acrylic or methacrylic acid (AA or MAA) monomer, or

iv) polymerization of at least one methyl methacrylate (MMA) monomer, of at least one monomer with a glass transition temperature below 30°C, such as butyl methacrylate (BMA), butyl acrylate (BA) or 2-ethylhexyl acrylate (2-EHA), and optionally of at least one acrylic acid (AA) or methacrylic acid (MAA) monomer.

A composition according to the invention preferably comprises at least one poly(meth)acrylate film-forming polymer of type ii) obtained by polymerization of at least one methyl methacrylate (MMA) monomer, of at least one monomer with a glass transition temperature below 30°C, such as butyl methacrylate (BMA), butyl acrylate (BA) or 2-ethylhexyl acrylate (2-EHA), and optionally of at least one acrylic acid (AA) or methacrylic acid (MAA) monomer.
Such a poly(meth)acrylate film-forming polymer of type ii) preferably corresponds to formula (XII) below:

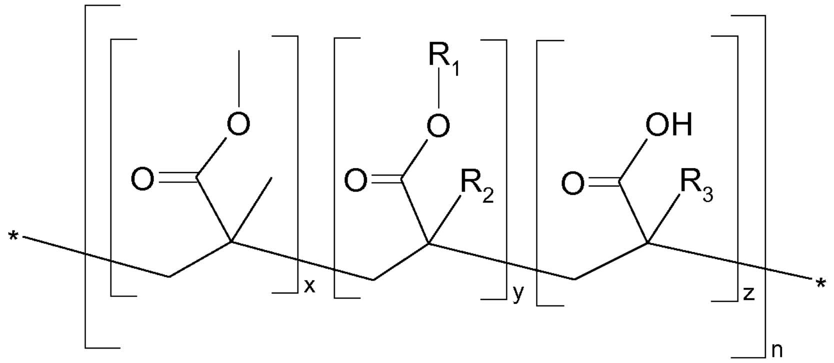

Formula (XII)

in which formula (XII):

- $R_1$, $R_2$ and $R_3$, which may be identical or different, represent a hydrogen atom or a $C_1$-$C_{10}$ alkyl group, $R_1$ preferably representing a $C_4$-$C_{10}$ alkyl group, and $R_2$ and $R_3$ preferably representing a hydrogen atom or a methyl group,
- x and y, which may be identical or different, represent an integer between 1 and 100,
- z represents an integer between 0 and 100,
- n represents an integer between 1 and 1000.

As a variant or additionally, a film-forming polymer that is suitable for the invention can be chosen from polysaccharides and polysaccharide derivatives, such as derivatives of cellulose or of guar gum. A preferential polysaccharide derivative suitable for the invention may be nitrocellulose or a polysaccharide ester or alkyl ether.

The term "polysaccharide ester or alkyl ether" denotes a polysaccharide made up of repeat units comprising at least two identical or different rings and having a degree of substitution per saccharide unit of between 1.9 and 3, preferably between 2.2 and 2.9 and more particularly between 2.4 and 2.8. The term "substitution" denotes the functionalization of the hydroxyl groups to give ester and/or alkyl ether functions, and/or the functionalization of the carboxylic groups to give ester functions.

In other words, it may be a polysaccharide, partially or totally substituted with ester and/or alkyl ether groups. Preferably, the hydroxyl groups may be substituted with ester and/or alkyl ether functions of $C_2$-$C_4$.

Mention may in particular be made of cellulose esters (such as cellulose acetobutyrates or cellulose acetopropionates), cellulose alkyl ethers (for instance ethylcelluloses), and ethyl guars.

A film-forming polymer that is suitable for the invention can be chosen from synthetic polymers such as polyurethanes, acrylic polymers, vinyl polymers, polyvinyl butyrals, alkyd resins and ketone/aldehyde resins, resins derived from aldehyde condensation products, such as arylsulfonamide-formaldehyde resins, for instance toluenesulfonamide-formaldehyde resin, arylsulfonamide-epoxy resins or ethyl tosylamide resins.

A film-forming polymer that is suitable for the invention can also be chosen from polymers of natural origin, such as plant resins, such as dammar resins, elemi resins, copal resins, and benzoin; gums such as shellac, sandarac gum and mastic gum.

Use may in particular be made, as film-forming polymers, of the toluenesulfonamide/formaldehyde resins Ketjentflex MS80 from the company Akzo or Santolite MHP or Santolite MS 80 from the company Faconnier or Resimpol 80 from the company Pan Americana, the alkyd resin Beckosol ODE 230-70-E from the company Dainippon, the acrylic resin Acryloid B66 from the company Rohm & Haas, the polyurethane resin Trixene PR 4127 from the company Baxenden or the acetophenone/formaldehyde resin sold under the reference Synthetic Resin SK by Degussa.

According to one particular embodiment, the film-forming polymer is chosen from the group consisting of polysaccharides and polysaccharide derivatives, preferably from nitrocellulose and polysaccharide ethers and esters, in particular of $C_2$-$C_4$, and more preferentially from cellulose acetobutyrates, cellulose acetopropionates, ethylcelluloses, ethyl guars, and mixtures thereof.

According to one embodiment, the film-forming polymer is chosen from the group consisting of nitrocellulose, cellulose acetopropionate, cellulose acetobutyrate, and (meth)acrylate homopolymers and copolymers, and mixtures thereof.

According to one embodiment, the compositions of the invention comprise at least one film-forming polymer chosen from nitrocellulose.

Preferably, a composition according to the invention comprises at least one film-forming polymer chosen from at least one polyacrylate compound of formula (XII).

According to this particular embodiment, in the second composition, the ratio between the weight of the film-forming polymer(s) and the photo-crosslinkable compounds, in particular the sum of the respective weights of the photo-crosslinkable compounds, is less than or equal to 1 and preferentially between 0.001 and 0.1.

***Volatile solvent(s)***

The second compositions according to the invention can comprise at least one volatile solvent as previously defined.
According to one particular embodiment, a second composition is free of volatile solvent(s).
According to another preferred embodiment, a second composition comprises a total content of solvent(s) less than or equal to 20% by weight, preferably between 0 and 10% by weight, relative to the total weight of the second composition.

***Photoinitiator(s)***

A second composition of a kit according to the invention also advantageously comprises at least one photoinitiator.
It can comprise a single photoinitiator or a mixture of several photoinitiators, preferably a single photoinitiator.
The photoinitiators that can be used in the second composition of the kit according to the invention are known in the art and are described, for example, in "Les photoinitiateurs dans la reticulation des revetements" ["Photoinitiators in the crosslinking of coatings"], G. Li Bassi, Double Liaison - Chimie des Peintures, no. 361, November 1985, pp. 34-41; "Applications industrielles de la polymériation photoinduite" ["Industrial applications of photoinduced polymerization"], Henri Strub, L'Actualité Chimique, February 2000, pp. 5-13; and "Photopolymères: considerations théoriques et reaction de prise" ["Photopolymers: theoretical considerations and setting reaction"], Marc, J.M. Abadie, Double Liaison - Chimie des Peintures, no. 435-436, 1992, pp. 28-34.
These photoinitiators encompass:

- α-hydroxy ketones, sold, for example, under the names Darocur® 1173 and 4265, Irgacure® 184, 2959, and 500 by the company BASF, and Additol® CPK by the company Cytec,
- α-amino ketones, sold, for example, under the names Irgacure® 907 and 369 by the company BASF,
- aromatic ketones, sold, for example, under the name Esacure® TZT by Lamberti. Mention may also be made of the thioxanthones sold, for example, under the name Esacure® ITX by Lamberti, and quinones. These aromatic ketones usually require the presence of a hydrogen-donating compound such as tertiary amines and in particular alkanolamines. Mention may in particular be made of the tertiary amine Esacure® EDB sold by the company Lamberti.
- α-dicarbonyl derivatives, the most common representative of which is benzyl dimethyl ketal, sold under the name Irgacure® 651 by BASF. Other commercial products are sold by the company Lamberti under the name Esacure® KB1, and
- acylphosphine oxides such as, for example, the bis-acylphosphine oxides (BAPOs) sold, for example, under the names Irgacure® 819, 1700 and 1800 and Darocur® 4265, Lucirin® TPO and Lucirin® TPO-L by the company BASF.

Preferably, the photoinitiator is chosen from the group consisting of α-hydroxy ketones, α-amino ketones, aromatic ketones preferably combined with a hydrogen-donating compound, aromatic α-diketones and acylphosphine oxides, and mixtures thereof.
An acylphosphine oxide is preferably used in the second photo-crosslinkable composition of the invention.
By way of photoinitiator, mention may be made of Lucirin® TPO-L (BASF).
The total content of photoinitiator(s) depends on a large number of factors such as, for example, the reactivity of the various components of the mixture, the presence of a colouring agent or colouring agents, the intensity of the light source or the exposure time.
In order to obtain the desired properties, the photoinitiator(s) is (are) preferably present in the second composition in a total content greater than or equal to 0.1% by weight, relative to the total weight of the second photo-crosslinkable composition, preferably ranging from 0.2% to 5% by weight, relative to the total weight of the second photo-crosslinkable composition.

***Other constituents***

A second composition of a kit according to the invention can also contain adjuvants, or additives, chosen in particular from stabilizers, colouring agents such as pigments, plasticizers, coalescers, preservatives, thickeners, fragrances, cosmetic nail care active agents, spreading agents, antifoams and dispersants.

Needless to say, those skilled in the art will take care to choose these optional adjuvants and additives such that the advantageous properties of the composition according to the invention are not, or are virtually not, adversely affected by the envisaged addition.

When the composition comprises colouring agents, the absorption spectrum of the colouring agents used should in particular be adapted to that of the photoinitiators, or conversely the absorption spectrum of the photoinitiators to that of the colouring agents used, in order to avoid these two types of compounds absorbing light at the same wavelengths. This is because the absorption of light by the colouring agents would render almost totally ineffective the photoinitiators present beyond a certain depth of the coating.

Preferably, the second composition of the invention is transparent.

As used herein, the term "transparent" means that the composition has a HAZEBYK index of less than 5 as measured with a KYKHAZEGLOSS brilliance meter.

According to one embodiment, the second composition of the invention also comprises a colouring agent chosen from the group consisting of soluble dyes, pigments, nacres and glitter flakes.

The colouring agent(s) can be present in the second composition in a total content greater than or equal to 0.1% by weight, relative to the total weight of the coat, preferably ranging from 0.1% to 5%, advantageously from 0.2% to 1% by weight, relative to the total weight of the second composition.

The term "soluble dyes" should be understood as meaning organic, inorganic or organometallic compounds which are soluble in the composition of the invention and intended to colour said composition.

The dyes are, for example, Sudan red, DC red 17, DC green 6, β-carotene, soybean oil, Sudan brown, DC yellow 11, DC violet 2, DC orange 5 and quinoline yellow.

The term "pigments" should be understood as meaning white or coloured and inorganic or organic particles of any shape which are insoluble in the composition of the invention and which are intended to colour said composition.

The term "nacres" should be understood as meaning iridescent particles of any shape, in particular produced by certain molluscs in their shell, or else synthesized.

The pigments may be white or coloured, and inorganic and/or organic. Among the inorganic pigments that may be mentioned are titanium dioxide, optionally surface-treated, zirconium oxide or cerium oxide, and also zinc oxide, iron (black, yellow or red) oxide or chromium oxide, manganese violet, ultramarine blue, chromium hydrate and ferric blue, and metal powders, for instance aluminium powder and copper powder.

Among the organic pigments that may be mentioned are carbon black, pigments of D & C type and lakes based on cochineal carmine or on barium, strontium, calcium or aluminium.

Mention may also be made of effect pigments, such as particles comprising an organic or inorganic and natural or synthetic substrate, for example glass, acrylic resins, polyester, polyurethane, polyethylene terephthalate, ceramics or aluminas, which may or may not be covered with metal substances, such as aluminium, gold, copper or bronze, or with metal oxides, such as titanium dioxide, iron oxide or chromium oxide, or with inorganic or organic pigments, and mixtures thereof.

The nacreous pigments can be chosen from white nacreous pigments, such as mica covered with titanium or with bismuth oxychloride, coloured nacreous pigments, such as titanium mica covered with iron oxides, titanium mica covered with in particular ferric blue or with chromium oxide, or titanium mica covered with an organic pigment of the abovementioned type, and nacreous pigments based on bismuth oxychloride.

Use may also be made of pigments with goniochromatic properties, in particular liquid crystal or multilayer pigments.

Optical brighteners or fibres optionally coated with optical brighteners can also be used.

The second compositions of a kit according to the invention can additionally comprise one or more fillers, in particular in a content ranging from 0.01% to 50% by weight, relative to the total weight of the composition, preferably ranging from 0.01% to 30% by weight.

The term "fillers" should be understood as meaning colourless or white, inorganic or synthetic particles of any shape, which are insoluble in the medium of the composition, irrespective of the temperature at which the composition is manufactured. These fillers serve in particular to modify the rheology or the texture of the composition.

The fillers may be inorganic or organic and of any shape, platelet-shaped, spherical or oblong, irrespective of the crystallographic form (for example lamellar, cubic, hexagonal, orthorhombic, etc.). Mention may be made of talc, mica, silica, kaolin, polyamide (Nylon®) powder (Orgasol® from Atochem), poly-p-alanine powder and polyethylene powder, powders of tetrafluoroethylene polymers (Teflon®), lauroyllysine, starch, boron nitride, hollow polymeric microspheres such as those of polyvinylidene chloride/acrylonitrile, for instance Expancel® (Nobel Industrie) or of acrylic acid copolymers (Polytrap® from the company Dow Corning) and silicone resin microbeads (for example Tospearls® from Toshiba), elastomeric polyorganosiloxane particles, precipitated calcium carbonate, magnesium carbonate, magnesium hydrogen carbonate, hydroxyapatite, hollow silica microspheres (Silica Beads® from Maprecos), glass or ceramic microcapsules, and metal soaps derived from organic carboxylic acids containing from 8 to 22 carbon atoms and preferably from 12 to 18 carbon atoms, for example zinc stearate, magnesium stearate or lithium stearate, zinc laurate or magnesium

myristate.

***USES OF THE KIT***

According to one embodiment, the kit according to the invention is intended to be applied to the nails and/or false nails, preferably before making up and/or caring for the nails and/or false nails, more preferentially for making up the nails and/or false nails.

In particular, the first and second compositions according to the invention are intended to be used as photo-crosslinkable nail varnish.

Preferably, the first composition is intended to be applied directly to the nails and/or false nails as a base coat or coating. Such a base coating then constitutes a first coating for at least one second photo-crosslinkable composition according to the invention.

The present invention also relates to a method for making up and/or caring for the nails and/or false nails, consisting in applying, to a nail and/or a false nail, a first photo-crosslinkable composition according to the invention followed by a second photo-crosslinkable composition according to the invention.

The radiation suitable for crosslinking the photo-crosslinkable composition of the present invention has a wavelength between 210 and 600 nm, preferably between 250 and 420 nm, preferably between 350 and 410 nm. The use of lasers may also be envisaged.

In one preferred embodiment of the invention, use is made of an LED lamp or a UV lamp and in particular a mercury-vapour lamp, the mercury optionally being doped with other elements, such as gallium, making it possible to modify the emission spectrum of the light source.

The radiation-exposure time of each deposited photo-crosslinkable coating depends on various factors such as the chemical nature and the content of the reactive components or else the desired crosslinking density.

For nail varnishes, it will generally be sought to obtain satisfactory results for an exposure time between 10 seconds and 10 minutes, preferably between 30 seconds and 5 minutes.

Such a method can use a UV lamp with a power of approximately 36 W.

Preferably, the thickness after drying of the first coating is less than or equal to 100 μm and preferably less than or equal to 75 μm.

Preferably, the thickness after drying of the second coating is less than or equal to 250 μm and preferably less than or equal to 200 μm.

At the end of the final crosslinking step, the coating deposited on the nail or the false nail can exhibit a tacky coat at its surface, requiring cleaning of the crosslinked coating using, for example, a solvent such as isopropanol.

The present invention also relates to a method for removing make up from the nails and/or false nails, comprising the application of a makeup-removal composition, such as a customary dissolving agent described above, to a nail or a false nail coated with at least one coat obtained by crosslinking a coat of first composition of the kit according to the invention and with at least one coat obtained by crosslinking a coat of second composition of the kit according to the invention, via which said crosslinked coats are removed.

The first coating, or base coating, obtained by application of the first composition of the kit in accordance with the invention, is coated with a second coating obtained by application of the second composition of the kit in accordance with the invention. In particular, this second coating is chosen from a top coating or a coloured coating. More particularly, the first coating can be coated with a coloured coating as second coating, and the second coating can itself be coated with a top coating as third coating. Preferably, each coating consists of a respective photo-crosslinkable composition and is the subject of photo-crosslinking according to the conditions set out above.

According to one particular embodiment, a method for coating the nails and/or false nails, in particular for making up and/or caring for the nails and/or false nails, comprises at least the following steps:

A) application, to a nail or a false nail, of a first composition of a kit in accordance with the invention, via which a first coating consisting of at least one coat of said photo-crosslinkable composition is deposited, this first coating being applied directly in contact with the nail or the false nail, and

B) exposure of the coated nail or false nail obtained at the end of step A) to a UV or visible light radiation,

C) application, to the first coating resulting from step A) and B), of a second composition of a kit in accordance with the invention, preferably distinct from the first composition, via which a second coating consisting of at least one coat of said second composition is deposited,

D) exposure of the coated nail or false nail obtained at the end of step C) to a UV or visible light radiation.

In such a method, the second coating is preferably a top coat, optionally free of colouring agent.

According to one embodiment, the method of the invention also comprises, before step B), a period of drying the coating deposited at the end of step A), the duration of which can range from 10 seconds to 10 minutes, typically

from 30 seconds to 5 minutes. Said drying is generally carried out in the open air and at ambient temperature.

The crosslinked coatings resulting from the crosslinking of step B) and D) exhibit a wear property over time, in terms of resistance to chipping and of gloss, which is significant and in particular on the scale of at least one week. They thus prove to be resistant to water, to rubbing and to impacts, and do not exhibit any significant wear or chipping during this period.

These coatings also have an ability to dissolve or to increase in volume and therefore in weight when they are brought into contact with a customary makeup-removing solvent. This ability to dissolve or to swell, shown by the crosslinked coating, is precisely advantageous for its removal when it is applied at the surface of a nail or of a false nail. Indeed, the coatings can be easily removed by simple makeup removal using a conventional dissolving agent

Thus, the first and second compositions of the kit according to the invention are advantageously removable using dissolving agents which are customary in the nail varnish field, and in particular using acetone and ethyl acetate, and mixtures thereof.

According to one particular embodiment, a method for coating the nails and/or false nails, in particular for making up and/or caring for the nails and/or false nails, comprises at least the following steps:

A) application, to a nail or a false nail, of a first composition of a kit in accordance with the invention, via which a first coating consisting of at least one coat of said photo-crosslinkable composition is deposited, this first coating being applied directly in contact with the nail or the false nail, and

B) exposure of the coated nail or false nail obtained at the end of step A) to a UV or visible light radiation,

C) application, to the first coating resulting from step A) and B), of a second composition of a kit in accordance with the invention, preferably distinct from the first composition, via which a second coating consisting of at least one coat of said second composition is deposited,

D) exposure of the coated nail or false nail obtained at the end of step C) to a UV or visible light radiation,

E) application, to the second coating resulting from step C) and D), of a third composition, preferably distinct from the first composition and from the second composition, via which a third coating consisting of at least one coat of said third composition is deposited,

F) exposure of the coated nail or false nail obtained at the end of step E) to a UV or visible light radiation.

In such a method, the second coating is preferably a coloured coat comprising at least one colouring agent and the third coating is preferably a top coat free of colouring agent.

According to one preferred embodiment, when a third coating of a third composition is applied, the second composition applied as second coating comprises at least one colouring agent. According to one particularly preferred embodiment, the second coating corresponds to one or preferably more coloured coats, which are preferably identical, such as two, comprising at least one colouring agent, and the third coating is preferably a top coat free of colouring agent.

According to one preferred embodiment, steps C) and D) are repeated in total twice, the second composition being applied a first time and exposed to a UV or visible light radiation, then being applied a second time and exposed to a UV or visible light radiation, steps A) and B), like E) and F), preferably being respectively carried out only once.

A subject of the present invention is also a kit comprising:

- a first photo-crosslinkable cosmetic composition according to the invention,
- a second photo-crosslinkable cosmetic composition according to the invention,
- an abrasive material having a particle size of greater than or equal to 200, preferably less than 300, advantageously between 220 and 280, and
- an LED lamp or a UV lamp.

A subject of the present invention is also a method for coating a nail and/or false nail, comprising the following steps:

i) rubbing the surface of a nail or of the false nail with an abrasive material having a particle size of greater than or equal to 200, preferably less than 300, advantageously between 220 and 280,

ii) applying a first photo-crosslinkable cosmetic composition according to the invention to the surface of the nail or of false nails which has been rubbed following step i), in order to deposit a coat consisting of at least one coat of said first composition,

iii) exposing the coated nail or false nails obtained following step ii) to an LED lamp or a UV lamp, such that photo-crosslinking is carried out so as to obtain a crosslinked coat,

iv) applying, to the nail or false nail coated with the crosslinked coat, obtained following step iii), a second photo-crosslinkable composition according to the invention, in order to deposit a coat consisting of at least one coat of said second composition, and

v) exposing the coated nail or false nails obtained after step iv) to an LED lamp or a UV lamp, such that photo-crosslinking is carried out so as to obtain a crosslinked coat.

The rubbing step is carried out for less than 10 seconds, preferably less than 5 seconds, for example for approximately 3 seconds.

The weight percentages given in this application can be categorized as the percentage by weight of dry matter of the compounds used, unless otherwise expressly mentioned.

The invention will be better understood on reading the following description, given solely by way of example.

**EXAMPLE:**

According to a first exemplary embodiment, the following first composition was prepared:

| Ingredients of the base coating composition | % Content |
|---|---|
| PYROMELLITIC DIMETHACRYLATE (PMDM X-865-0000 - ESSTECH, Inc.) | 7.5 |
| PEG-400 Urethane dimethacrylate (X-726-0000 - ESSTECH, Inc.) | 16 |
| TETRAHYDROFURFURYL METHACRYLATE (X-958-7446 - ESSTECH, Inc.) | 1.5 |
| METHYL METHACRYLATE (MMA) / BUTYL METHACRYLATE (BMA) COPOLYMER (PARALOID B 66 100% from DOW CHEMICAL) | 7 |
| Nitrocellulose containing 30% of isopropyl alcohol (viscosity: E22 - 1/2s) (IDYL E35 TX IPA 30% from BERGERAC - SNPE) | 7 |
| Ethyl acetate | 21.65 |
| Propyl acetate | 10 |
| Butyl acetate | 25 |
| Photoinitiator ethyl (2,4,6-trimethylbenzoyl) phenylphosphinate (Lucirin TPO-L - BASF) | 4 |
| BHT (DI-(TERT-BUTYL)-4-HYDROXYTOLUENE - NIPANOX BHT from CLARIANT) | 0.35 |

The ingredients of the composition are introduced into an opaque flask and stirred away from the light with a laboratory mixer of the Rayneri brand until a homogeneous mixture is obtained. A sheet of aluminium will have been placed over the container beforehand in order to prevent evaporation of the solvents.

On a nail roughened beforehand for less than 5 seconds using a file with a particle size of 280, the first composition described above was applied to said nail so as to form a base coating or base coat.

After application, the nail is placed under a 36 W UV lamp for 3 minutes in order to crosslink the composition so as to form a film.

The following top composition is then prepared:

| Ingredients of the top composition | % Content |
|---|---|
| TETRAHYDROFURFURYL METHACRYLATE (X-958-7446 - ESSTECH, Inc.) | 20 |
| Isophorone Urethane Dimethacrylate (X-851-1066 - ESSTECH, Inc.) | 15 |
| PEG-400 Urethane dimethacrylate (X-726-0000 - ESSTECH, Inc.) | 60 |
| METHYL METHACRYLATE (MMA) / BUTYL METHACRYLATE (BMA) COPOLYMER (PARALOID B 66 100% from DOW CHEMICAL) | 1 |
| Photoinitiator ethyl (2,4,6-trimethylbenzoyl) phenylphosphinate (Lucirin TPO-L - BASF) | 4 |

The ingredients of the composition are introduced into an opaque flask and stirred away from the light with a

laboratory mixer of the Rayneri brand until a homogeneous mixture is obtained. A sheet of aluminium will have been placed over the container beforehand in order to prevent evaporation of the solvents.

This composition is applied to the first coating (base coating) in the form of one or more coats so as to form a top coating.

After application of each coat, in the case just one, the nail is placed under a 36 W UV lamp for 3 minutes in order to crosslink the composition so as to form a film, this operation being repeated for each coat applied.

After having crosslinked the final coat, the surface is cleaned with cotton wool soaked in isopropanol in order to remove the tacky coat.

According to another exemplary embodiment, prior to this top coating, a coloured composition similar to this top composition, except that this coloured composition additionally comprises one or more colouring agent(s), is applied. Such a coloured composition has the following composition:

| Ingredients of the coloured composition | % Content |
|---|---|
| TETRAHYDROFURFURYL METHACRYLATE (X-958-7446 - ESSTECH, Inc.) | 20 |
| Isophorone Urethane Dimethacrylate (X-851-1066 - ESSTECH, Inc.) | 15 |
| PEG-400 Urethane dimethacrylate (X-726-0000 - ESSTECH, Inc.) | 59 |
| METHYL METHACRYLATE (MMA) / BUTYL METHACRYLATE (BMA) COPOLYMER (PARALOID B 66 100% from DOW CHEMICAL) | 1 |
| Photoinitiator ethyl (2,4,6-trimethylbenzoyl) phenylphosphinate (Lucirin TPO-L - BASF) | 4 |
| Pigment | 1 |

The ingredients of the composition are introduced into an opaque flask and stirred away from the light with a laboratory mixer of the Rayneri brand until a homogeneous mixture is obtained. A sheet of aluminium will have been placed over the container beforehand in order to prevent evaporation of the solvents.

After application of the base coating, one or more coats of the coloured composition, in the case two coats, was (were) applied to the base coating. After the application of each coat, the nail is placed under a 36 W UV lamp for 3 minutes in order to crosslink the composition so as to form a coloured coating in the form of a film.

After application of the coloured coating and photo-crosslinking of this coating in the form of a film, the top composition previously described is then applied to this coloured coating in the form of one or more coats, in the case a single coat. After the application of this coat, the nail is placed under a 36 W UV lamp for 3 minutes in order to crosslink the composition so as to form a top coating in the form of a film.

After having crosslinked the final coat, the surface is cleaned with cotton wool soaked in isopropanol in order to remove the tacky coat. In the two embodiments according to the invention evaluated, each comprising a base coating comprising a photo-crosslinkable composition in accordance with the invention, a varnish exhibiting a good wear property on the nail is thus obtained. To do this, the wear property was assessed by simple observation with the naked eye after 14 days following its application. This wear property performance is produced with only a very slight roughening of the nail before application of said compositions, making it possible to avoid the conventional invasive method of attaching a photo-crosslinkable composition to the nails by sanding the surface of the nail, while at the same time preserving performance levels equivalent to or even better than products currently on the market.

Furthermore, the ingredients used in the compositions according to the invention make it possible to have, after photo-crosslinking of the film, an extremely low content of extractable compounds comprising reactive (meth)acrylate functions with potentially sensitizing effects.

The varnish can then be completely removed after having been placed in contact with acetone for 15 minutes, this time again therefore without a conventional invasive method using a metal tool, an electric sander, or an abrasive file by rubbing against the surface of the made-up nail in order to remove the composition.

Throughout the application, the wording "comprising one" or "including one" means "comprising at least one" or "including at least one", unless otherwise specified.

## Claims

**1.** Kit for coating a nail or false nail, and more particularly for making up a nail or false nail, comprising:

- a first photo-crosslinkable composition comprising, in a physiologically acceptable medium, at least one photo-crosslinkable compound a) comprising at least two (ALK)acrylate functions, wherein ALK represents a $C_1$-$C_6$, preferably $C_1$-$C_2$, more preferably $C_1$, alkyl group, such as $CH_3$, and at least one carboxylic acid function,
- a second photo-crosslinkable composition comprising, in a physiologically acceptable medium:

• at least one photo-crosslinkable compound b) comprising at least one (poiy)urethane poly(ALK)acrylate compound wherein ALK represents a $C_1$-$C_6$, preferably $C_1$-$C_2$, more preferably $C_1$, alkyl group, such as $CH_3$, preferably polyurethane di(meth)acrylate compound, more preferentially polyurethane dimethacrylate compound, the photo-crosslinkable compound(s) b) comprising a (poly)oxyalkylene unit, in particular comprising a (poly)oxyethylene unit, preferably comprising from 1 to 100 oxyalkylene units, preferably from 5 to 50 oxyalkylene units, and preferentially approximately 8 to 10 oxyalkylene units, preferably identical to that contained in the first composition,
• at least one photo-crosslinkable compound c) comprising at least two carbamate units obtained by reaction with at least one diisocyanate of isophorone diisocyanate type, the compound(s) c) correspond(s) to formula (XIII) below:

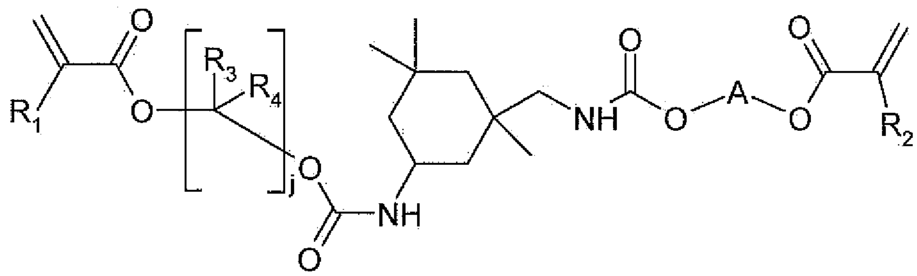

Formula (XIII)

in which formula (XIII):

- $R_1$, $R_2$, $R_3$ and $R_4$, which may be identical or different, represent a hydrogen atom or a $C_1$-$C_{10}$ alkyl chain, preferably a hydrogen atom or a methyl group,
- j is between 1 and 10, preferably equal to 2,
- A represents a $C_1$-$C_{10}$ alkyl group, or a polyurethane comprising from 2 to 20 carbamate units.

• at least one (ALK)acrylate monomer, wherein ALK represents a $C_1$-$C_6$, preferably $C_1$-$C_2$, more preferably $C_1$, alkyl group, such as $CH_3$, preferably (meth)acrylate monomer, more preferentially tetrahydrofurfuryl methacrylate.

**2.** Kit according to Claim 1, in which the compound(s) a) correspond(s) to formula (I) below:

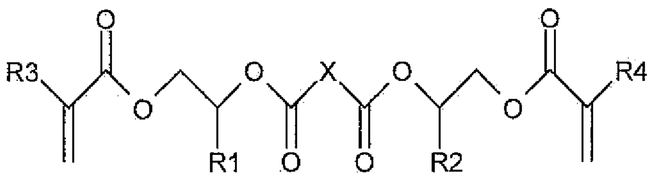

(I)

in which formula (I):

- R1 and R2, which may be identical or different, represent a hydrogen atom, or a methyl group, or a group of formula (II) below:

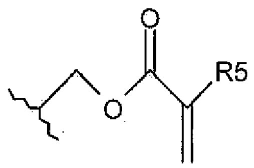

(II)

R5 representing a hydrogen atom or a methyl group;

- R3 and R4, which may be identical or different, represent a hydrogen atom or a methyl group;
- X represents a radical corresponding to one of formula (III), (IV), (V), (VI), (VII), (VIII), (IX) or (X) below:

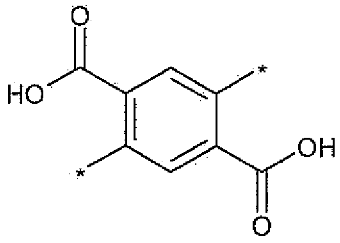

(III)

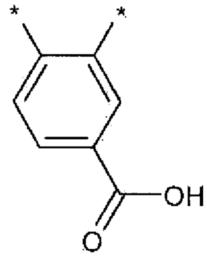

(IV)

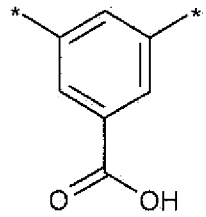

(V)

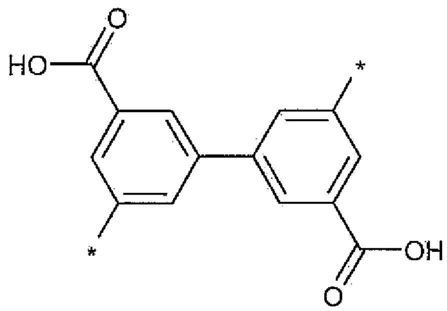

(VI)

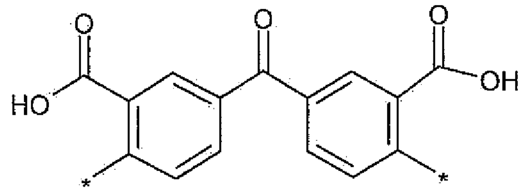

(VII)

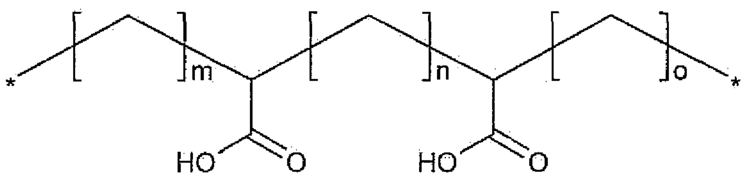

(VIII)

m, n and o, which may be identical or different, representing an integer between 0 and 10;

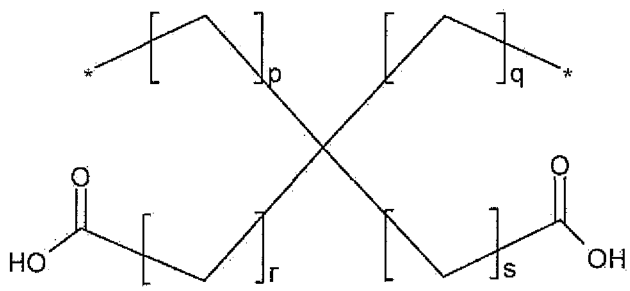

(IX)

p, q, r and s, which may be identical or different, representing an integer between 0 and 10;

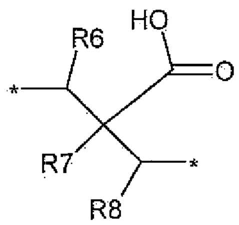

(X)

R6, R7 and R8, which may be identical or different, representing a hydrogen atom or a hydroxyl group.

**3.** Kit according to Claim 2, in which the compound(s) a) correspond(s) to formula (1-1) below:

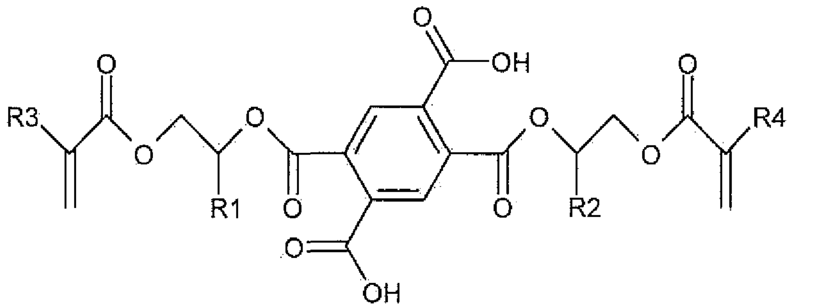

(I-1)

in which formula (1-1) R1, R2, R3 and R4 are as defined in Claim 2.

**4.** Kit according to Claim 3, in which R3 and R4 of formula (1-1) are methyl groups.

**5.** Kit according to Claim 3 or 4, in which R1 and R2 of formula (1-1) represent hydrogen atoms.

**6.** Kit according to any one of Claims 2 to 5, in which the compound(s) a), corresponding to formula (I), is (are) present in the first composition in a content greater than or equal to 10% by weight, relative to the weight of the total solids of the first composition, in particular ranging from 10% to 25% by weight, preferably from 15% to 20% by weight, relative to the weight of the total solids of the first composition.

**7.** Kit according to any one of the preceding claims, in which the first composition comprises at least one photo-crosslinkable compound b) comprising at least one (poly)urethane poly(ALK)acrylate compound, wherein ALK represents a $C_1$-$C_6$, preferably $C_1$-$C_2$, more preferably $C_1$, alkyl group, such as $CH_3$, preferably polyurethane di(meth)acrylate compound, more preferentially polyurethane dimethacrylate compound, the photo-crosslinkable compound(s) b) comprising a (poly)oxyalkylene unit, in particular comprising a (poly)oxyethylene unit, preferably comprising from 1 to 100 oxyalkylene units, preferably from 5 to 50 oxyalkylene units, and preferentially approximately 8 to 10 oxyalkylene units, preferably identical to that contained in the first composition.

**8.** Kit according to any one of the preceding claims, in which the compound(s) b) correspond(s) to formula (XI) below:

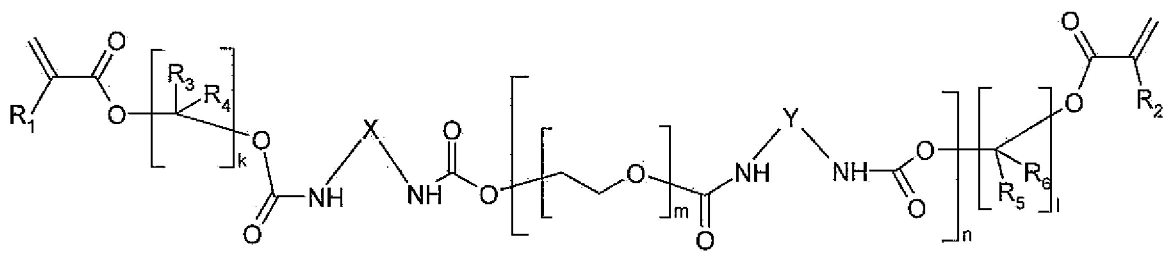

Formula (XI)

in which formula (XI):

- $R_1$, $R_2$, $R_3$, $R_4$, $R_5$ and $R_6$, which may be identical or different, represent a hydrogen atom or a $C_1$-$C_6$ alkyl chain, preferably a hydrogen atom or a methyl group,
- k and l, which may be identical or different, are between 1 and 10, preferably equal to 2,
- m is between 1 and 100, preferably between 5 and 50, preferably between approximately 8 and 10,
- n is between 1 and 10, preferably equal to 1,
- X and Y, which may be identical or different, represent a $C_1$-$C_{20}$ alkyl or cycloalkyl group.

**9.** Kit according to Claim 7 or 8, in which the compound(s) b) present in the first composition, in particular corresponding to formula (XI), is (are) present in a content greater than or equal to 20% by weight, relative to the total weight of the solids of the first composition, in particular ranging from 25% to 50% by weight, preferably from 30% to 50% by weight, relative to the weight of the total solids of the first composition.

**10.** Kit according to any one of the preceding claims, in which the compound(s) b) present in the second composition, in particular corresponding to formula (XI), is (are) present in a content greater than or equal to 10% by weight, relative to the total weight of the solids of the second composition, in particular ranging from 25% to 80% by weight, preferably from 50% to 70% by weight, relative to the weight of the total solids of the second composition.

**11.** Kit according to any one of the preceding claims, in which the compound(s) c), corresponding to formula (XIII), is (are) present in the second composition in a content greater than or equal to 5% by weight, relative to the total weight of the solids of the second composition, in particular ranging from 10% to 80% by weight, preferably from 15% to 70% by weight, relative to the weight of the total solids of the second composition.

**12.** Kit according to any one of the preceding claims, in which the first composition and the second composition comprise at least one film-forming polymer, preferably chosen from the group consisting of poly(meth)acrylates, polysaccharides and derivatives, and of a mixture thereof, preferably a mixture thereof.

**13.** Kit according to any one of the preceding claims, in which the first composition comprises at least one film-forming polymer chosen from the group consisting of poly(meth)acrylates, and preferably a mixture of poly(meth)acrylate(s) and of polysaccharide(s) and derivatives.

**14.** Kit according to any one of the preceding claims, in which the second composition comprises at least one film-forming polymer chosen from the group of polysaccharides and derivatives.

**15.** Kit according to any one of Claims 12, 13 or 14 in which the film-forming polymer(s) comprise(s) at least one poly(meth)acrylate corresponding to formula (XII) below:

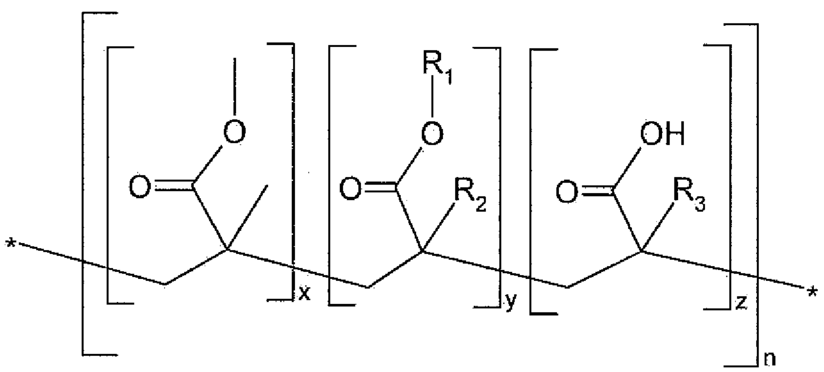

Formula (XII)

in which formula (XII):

- $R_1$, $R_2$ and $R_3$, which may be identical or different, represent a hydrogen atom or a $C_1$-$C_{10}$ alkyl group, $R_1$ preferably representing a $C_4$-$C_{10}$ alkyl group, and $R_2$ and $R_3$ preferably representing a hydrogen atom or a methyl group,
- x and y, which may be identical or different, represent an integer between 1 and 100,
- z represents an integer between 0 and 100,

- n represents an integer between 1 and 1000.

**16.** Kit according to any one of Claims 12 to 15, in which the polysaccharide(s) and polysaccharide derivative(s) is (are) chosen from nitrocellulose and ethers and esters of polysaccharides, in particular of $C_2$-$C_4$, in particular from cellulose acetobutyrates, cellulose acetopropionates, ethylcelluloses, ethyl guars, and mixtures thereof, more preferentially chosen from nitrocellulose.

**17.** Kit according to any one of Claims 12 to 16, in which the film-forming polymer(s) is (are) present in the first composition in a total content greater than or equal to 20% by weight, relative to the total weight of the solids of the first composition, preferably from 25% to 40% by weight, relative to the weight of the total solids of the first composition.

**18.** Kit according to any one of Claims 12 to 17 in which the film-forming polymer(s) is (are) present in the second composition in a total content greater than or equal to 0.05% by weight, relative to the total weight of the solids of the second composition, in particular ranging from 0.1% to 10% by weight, preferably from 0.2% to 5% by weight, relative to the weight of the total solids of the second composition.

**19.** Kit according to any one of the preceding claims, in which the first composition comprises at least one volatile solvent, preferably at least one polar volatile solvent advantageously chosen from the group consisting of $C_3$-$C_6$ esters and ketones and mixtures thereof.

**20.** Kit according to Claim 19, in which the volatile solvent(s) is (are) present in the first composition in a total content greater than or equal to 30% by weight, relative to the total weight of the composition, in particular ranging from 50% to 70%, relative to the total weight of the first composition.

**21.** Kit according to any one of the preceding claims, in which the first composition and the second composition comprise at least one photoinitiator, the photoinitiator preferably being chosen from the group consisting of $\alpha$-hydroxy ketones, $\alpha$-amino ketones, aromatic ketones preferably combined with a hydrogen-donating compound, aromatic $\alpha$-diketones and acylphosphine oxides, and mixtures thereof, advantageously from the group consisting of acylphosphine oxides.

**22.** Kit according to any one of the preceding claims, in which the first composition comprises at least one (ALK)acrylate monomer, wherein ALK represents a $C_1$-$C_6$, preferably $C_1$-$C_2$, more preferably $C_1$, alkyl group, such as $CH_3$, preferably (meth)acrylate monomer, more preferentially tetrahydrofurfuryl methacrylate.

**23.** Kit according to the preceding claim, in which the (ALK)acrylate monomer(s) wherein ALK represents a $C_1$-$C_6$, preferably $C_1$-$C_2$, more preferably $C_1$, alkyl group, such as $CH_3$, is (are) present in the first composition in a total content greater than or equal to 0.1% by weight, relative to the total weight of the solids of the first composition, in particular ranging from 0.2% to 10% by weight, preferably from 0.5% to 5% by weight, relative to the weight of the total solids of the first composition.

**24.** Kit according to any one of the preceding claims, in which the (ALK)acrylate monomer(s) wherein ALK represents a $C_1$-$C_6$, preferably $C_1$-$C_2$, more preferably $C_1$, alkyl group, such as $CH_3$, is (are) present in the second composition in a total content greater than or equal to 2% by weight, relative to the total weight of the solids of the second composition, in particular ranging from 5% to 40% by weight, preferably from 10% to 30% by weight, relative to the weight of the total solids of the second composition.

**25.** Method for coating the nails and/or false nails, in particular for making up and/or caring for the nails and/or false nails, comprising at least the following steps:

A) application, to a nail or a false nail, of a first photo-crosslinkable composition of the kit according to any one of Claims 1 to 23, via which a coating consisting of at least one coat of said first photo-crosslinkable composition is deposited,
B) exposure of the coated nail or false nail obtained at the end of step A) to a UV or visible light radiation,
C) application, to the first coating resulting from step A) and B), of a second composition of the kit according to any one of Claims 1 to 23, via which a second coating consisting of at least one coat of said second composition is deposited,
D) exposure of the coated nail or false nail obtained at the end of step C) to a UV or visible light radiation.

**26.** Method for coating the nails and/or false nails, in particular for making up and/or caring for the nails and/or false

nails, comprising at least the following steps:

A) application, to a nail or a false nail, of a first photo-crosslinkable composition of the kit according to any one of Claims 1 to 23, via which a coating consisting of at least one coat of said first photo-crosslinkable composition is deposited,
B) exposure of the coated nail or false nail obtained at the end of step A) to a UV or visible light radiation,
C) application, to the first coating resulting from step A) and B), of a second composition of the kit according to any one of Claims 1 to 23, via which a second coating consisting of at least one coat of said second composition is deposited,
D) exposure of the coated nail or false nail obtained at the end of step C) to a UV or visible light radiation,
E) application, to the second coating resulting from step C) and D), of a third composition, distinct from the first composition and from the second composition, via which a third coating consisting of at least one coat of said third composition is deposited,
F) exposure of the coated nail or false nail obtained at the end of step E) to a UV or visible light radiation.

**27.** Method for coating the nails and/or false nails according to Claim 26, in which, when a third coating of a third composition is applied, the second composition applied as second coating comprises at least one colouring agent.

**28.** Method for coating the nails and/or false nails according to Claim 26 or 27, in which steps C) and D) are repeated in total twice, the second composition being applied a first time and exposed to a UV or visible light radiation, then being applied a second time and exposed to a UV or visible light radiation, steps A) and B), like E) and F), preferably being respectively carried out only once.

## Patentansprüche

**1.** Kit zum Beschichten eines Nagels oder eines künstlichen Nagels und insbesondere zum Zurechtmachen eines Nagels oder eines künstlichen Nagels, umfassend:

- eine erste photovernetzbare Zusammensetzung, die in einem physiologisch unbedenklichen Medium mindestens eine photovernetzbare Verbindung a) mit mindestens zwei (ALK)acrylat-Funktionen, wobei ALK für eine $C_1$-$C_6$-Alkylgruppe, bevorzugt eine $C_1$-$C_2$-Alkylgruppe, weiter bevorzugt eine $C_1$-Alkylgruppe, wie $CH_3$, steht, und mindestens eine Carbonsäurefunktion umfasst,
- eine zweite photovernetzbare Zusammensetzung, die in einem physiologisch unbedenklichen Medium:

  • mindestens eine photovernetzbare Verbindung b), die mindestens eine (Poly)urethanpoly(ALK)-acrylat-Verbindung umfasst, wobei ALK für eine $C_1$-$C_6$-Alkylgruppe, bevorzugt eine $C_1$-$C_2$-Alkylgruppe, weiter bevorzugt eine $C_1$-Alkylgruppe, wie $CH_3$, steht, bevorzugt eine Polyurethandi(meth)acrylat-Verbindung, weiter bevorzugt eine Polyurethandimethacrylat-Verbindung, steht, wobei die photovernetzbare Verbindung bzw. die photovernetzbaren Verbindungen b) eine (Poly)oxyalkylen-Einheit, insbesondere eine (Poly)oxyethylen-Einheit, bevorzugt mit 1 bis 100 Oxyalkylen-Einheiten, bevorzugt 5 bis 50 Oxyalkylen-Einheiten und vorzugsweise ungefähr 8 bis 10 Oxyalkylen-Einheiten, umfasst bzw. umfassen, die bevorzugt mit der in der ersten Zusammensetzung enthaltenen Verbindung identisch ist,
  • mindestens eine photovernetzbare Zusammensetzung (c) mit mindestens zwei Carbamat-Einheiten, erhalten durch Reaktion mit mindestens einem Diisocyanat vom Isophorondiisocyanat-Typ, wobei die Verbindung bzw. die Verbindungen c) der nachstehenden Formel (XIII) entspricht bzw. entsprechen:

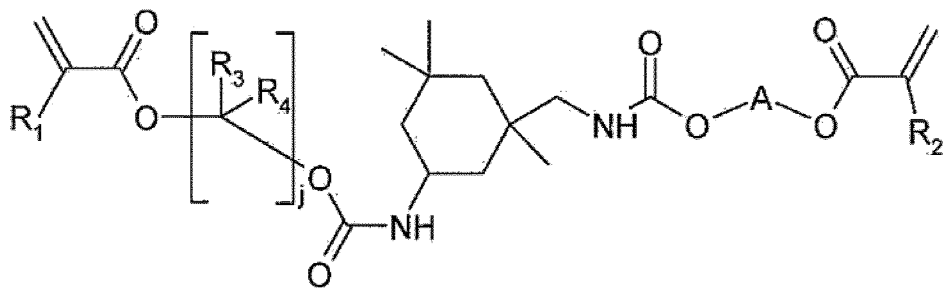

Formel (XIII)

wobei in der Formel (XIII):

- $R_1$, $R_2$, $R_3$ und $R_4$, die gleich oder verschieden sein können, für ein Wasserstoffatom oder eine $C_1$-$C_{10}$-Alkylkette, vorzugsweise ein Wasserstoffatom oder eine Methylgruppe, stehen,
- j zwischen 1 und 10 liegt und vorzugsweise gleich 2 ist,
- A für eine $C_1$-$C_{10}$-Alkylgruppe oder ein Polyurethan mit 2 bis 20 Carbamat-Einheiten steht,

• mindestens ein (ALK)acrylat-Monomer, wobei ALK für eine $C_1$-$C_6$-Alkylgruppe, bevorzugt eine $C_1$-$C_2$-Alkylgruppe, weiter bevorzugt eine $C_1$-Alkylgruppe, wie $CH_3$, steht, bevorzugt ein (Meth)acrylat-Monomer, weiter bevorzugt ein Tetrahydrofurfurylmethacrylat,
umfasst.

**2.** Kit nach Anspruch 1, in der die Verbindung bzw. die Verbindungen a) der nachstehenden Formel (I) entspricht bzw. entsprechen:

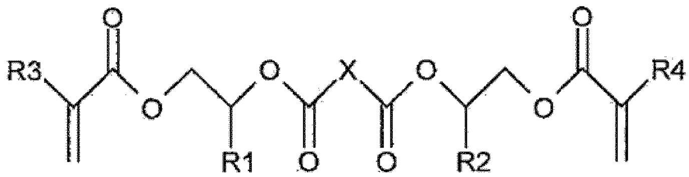

(I)

wobei in der Formel (I):

- R1 und R2, die gleich oder verschieden sein können, für ein Wasserstoffatom oder eine Methylgruppe oder eine Gruppe der nachstehenden Formel (II) stehen:

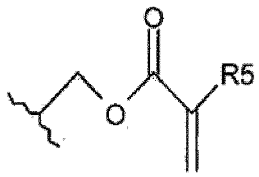

(II)

R5 für ein Wasserstoffatom oder eine Methylgruppe steht,
- R3 und R4, die gleich oder verschieden sein können, für ein Wasserstoffatom oder eine Methylgruppe stehen,
- X für einen Rest steht, der einer der nachstehenden Formeln (III), (IV), (V), (VI), (VII), (VIII), (IX) oder (X) entspricht:

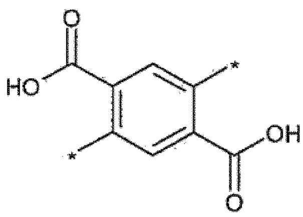

(III)

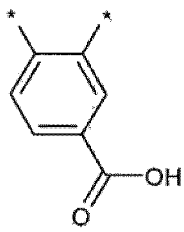

(IV)

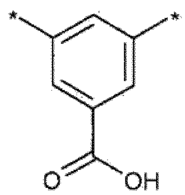

(V)

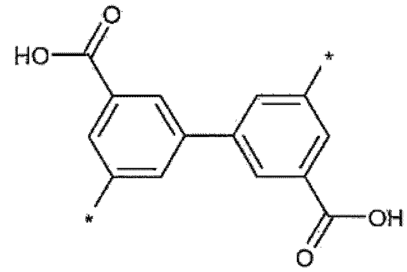

(VI)

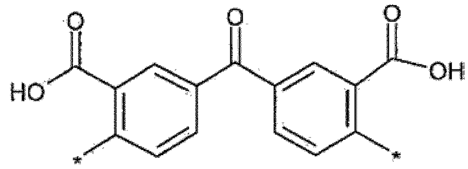

(VII)

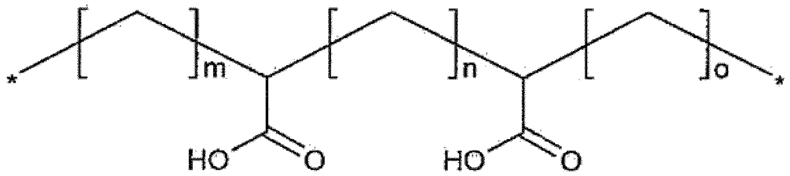

(VIII)

m, n und o, die gleich oder verschieden sein können, für eine ganze Zahl zwischen 0 und 10 stehen;

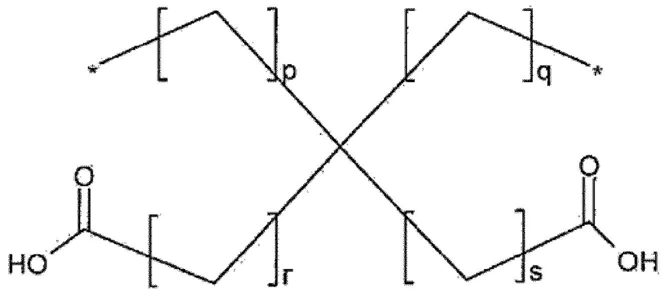

(IX)

p, q, r und s, die gleich oder verschieden sein können, für eine ganze Zahl zwischen 0 und 10 stehen;

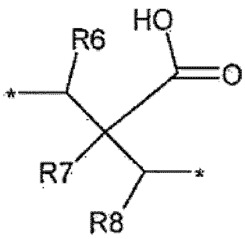

(X)

R6, R7 und R8, die gleich oder verschieden sein können, für ein Wasserstoffatom oder eine Hydroxylgruppe stehen.

**3.** Kit nach Anspruch 2, wobei die Verbindung bzw. Verbindungen a) der nachstehenden Formel (I-1) entspricht bzw. entsprechen:

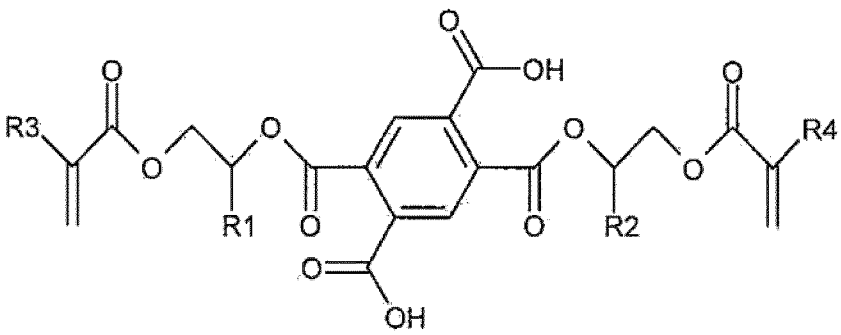

(I-1)

wobei in der Formel (I-1) R1, R2, R3 und R4 wie in Anspruch 2 definiert sind.

**4.** Kit nach Anspruch 3, wobei R3 und R4 der Formel (I-1) für Methylgruppen stehen.

**5.** Nach Anspruch 3 oder 4, wobei R1 und R2 der Formel (I-1) für Wasserstoffatome stehen.

**6.** Kit nach einem der Ansprüche 2 bis 5, wobei die Verbindung bzw. die Verbindungen a), die der Formel (I) entspricht bzw. entsprechen, in der ersten Zusammensetzung in einem Gehalt größer oder gleich 10 Gew.-%, bezogen auf das Gewicht der gesamten Feststoffe der ersten Zusammensetzung, insbesondere im Bereich von 10 bis 25 Gew.-%, bevorzugt von 15 bis 20 Gew.-%, bezogen auf das Gewicht der gesamten Feststoffe der ersten Zusammensetzung, vorliegt bzw. vorliegen.

**7.** Kit nach einem der vorhergehenden Ansprüche, wobei die erste Zusammensetzung mindestens eine photovernetzbare Verbindung b) umfasst, die mindestens eine (Poly)urethanpoly(ALK)acrylat-Verbindung umfasst, wobei ALK für eine $C_1$-$C_6$-Alkylgruppe, bevorzugt eine $C_1$-$C_2$-Alkylgruppe, weiter bevorzugt eine $C_1$-Alkylgruppe, wie $CH_3$, steht, bevorzugt eine Polyurethandi(meth)acrylat-Verbindung, weiter bevorzugt eine Polyurethandimethacrylat-Verbindung, steht, wobei die photovernetzbare Verbindung bzw. die photovernetzbaren Verbindungen b) eine (Poly)oxyalkylen-Einheit, insbesondere eine (Poly)oxyethylen-Einheit, bevorzugt mit 1 bis 100 Oxyalkylen-Einheiten, bevorzugt 5 bis 50 Oxyalkylen-Einheiten und vorzugsweise ungefähr 8 bis 10 Oxyalkylen-Einheiten, umfasst bzw. umfassen, die bevorzugt mit der in der ersten Zusammensetzung enthaltenen Verbindung identisch ist.

**8.** Kit nach einem der vorhergehenden Ansprüche, wobei die Verbindung bzw. die Verbindungen b) der nachstehenden Formel (XI) entspricht bzw. entsprechen:

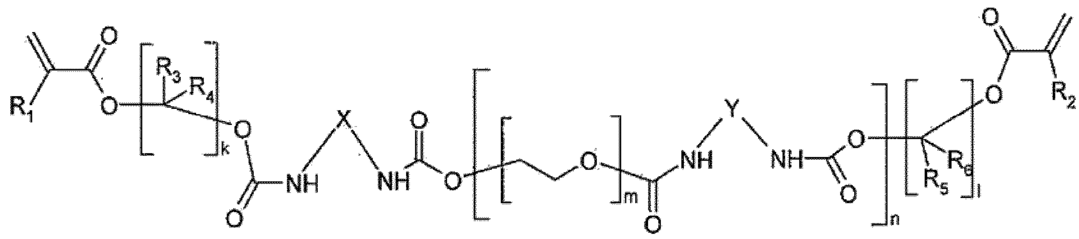

Formel (XI)

wobei in der Formel (XI):

- $R_1$, $R_2$, $R_3$, $R_4$, $R_5$ und $R_6$, die gleich oder verschieden sein können, für ein Wasserstoffatom oder eine $C_1$-$C_6$-Alkylkette, vorzugsweise ein Wasserstoffatom oder eine Methylgruppe, stehen,
- k und 1, die gleich oder verschieden sein können, zwischen 1 und 10 liegen und vorzugsweise gleich 2 sind,
- m zwischen 1 und 100, vorzugsweise zwischen 5 und 50, vorzugsweise zwischen ungefähr 8 und 10, liegt,
- n zwischen 1 und 10 liegt und vorzugsweise gleich 1 ist,
- X und Y, die gleich oder verschieden sein können, für eine $C_1$-$C_{20}$-Alkyl- oder Cycloalkylgruppe stehen.

**9.** Kit nach Anspruch 7 oder 8, wobei die Verbindung bzw. die Verbindungen b) in der ersten Zusammensetzung, die insbesondere der Formel (XI) entspricht bzw. entsprechen, in einem Gehalt größer oder gleich 20 Gew.-%, bezogen auf das Gesamtgewicht der Feststoffe der ersten Zusammensetzung, insbesondere im Bereich von 25 bis 50 Gew.-%, vorzugsweise von 30 bis 50 Gew.-%, bezogen auf das Gewicht der gesamten Feststoffe der ersten Zusammensetzung, vorliegt bzw. vorliegen.

**10.** Kit nach einem der vorhergehenden Ansprüche, wobei die Verbindung bzw. die Verbindungen b) in der zweiten Zusammensetzung, die insbesondere der Formel (XI) entspricht bzw. entsprechen, in einem Gehalt größer oder gleich 10 Gew.-%, bezogen auf das Gesamtgewicht der Feststoffe der zweiten Zusammensetzung, insbesondere im Bereich von 25 bis 80 Gew.-%, vorzugsweise von 50 bis 70 Gew.-%, bezogen auf das Gewicht der gesamten Feststoffe der zweiten Zusammensetzung, vorliegt bzw. vorliegen.

**11.** Kit nach einem der vorhergehenden Ansprüche, wobei die Verbindung bzw. die Verbindungen c), die der Formel (XIII) entspricht bzw. entsprechen, in der zweiten Zusammensetzung in einem Gehalt größer oder gleich 5 Gew.-

%, bezogen auf das Gesamtgewicht der Feststoffe der zweiten Zusammensetzung, insbesondere im Bereich von 10 bis 80 Gew.-%, vorzugsweise von 15 bis 70 Gew.-%, bezogen auf das Gewicht der gesamten Feststoffe der zweiten Zusammensetzung, vorliegt bzw. vorliegen.

**12.** Kit nach einem der vorhergehenden Ansprüche, wobei die erste Zusammensetzung und die zweite Zusammensetzung mindestens ein filmbildendes Polymer umfassen, das vorzugsweise aus der Gruppe bestehend aus Poly(meth)acrylaten, Polysacchariden und Derivaten und einer Mischung davon, vorzugsweise einer Mischung davon, ausgewählt ist.

**13.** Kit nach einem der vorhergehenden Ansprüche, wobei die erste Zusammensetzung mindestens ein filmbildendes Polymer umfasst, das vorzugsweise aus der Gruppe bestehend aus Poly(meth)acrylaten und vorzugsweise einer Mischung von Poly(meth)-acrylat(en) und von Polysaccharid(en) und Derivaten ausgewählt ist.

**14.** Kit nach einem der vorhergehenden Ansprüche, wobei die zweite Zusammensetzung mindestens ein filmbildendes Polymer umfasst, das aus der Gruppe bestehend aus Polysacchariden und Derivaten ausgewählt ist.

**15.** Kit nach einem der Ansprüche 12, 13 oder 14, wobei das filmbildende Polymer bzw. die filmbildenden Polymere mindestens ein Poly(meth)acrylat der nachstehenden Formel (XII) umfasst bzw. umfassen:

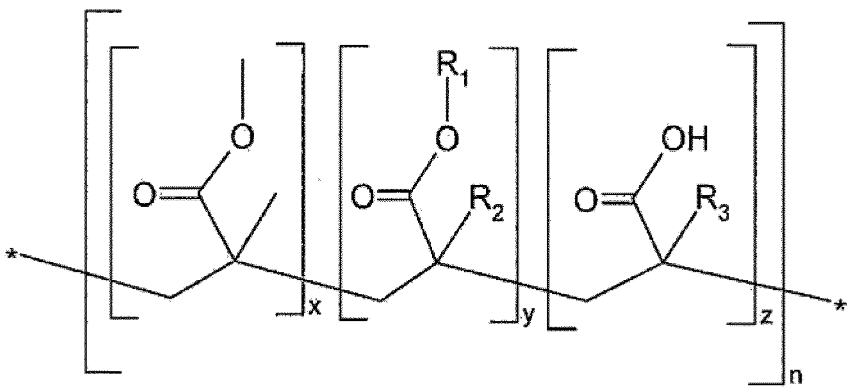

Formel (XII)

wobei in der Formel (XII):

- $R_1$, $R_2$ und $R_3$, die gleich oder verschieden sein können, für Wasserstoff oder eine $C_1$-$C_{10}$-Alkylgruppe stehen, wobei $R_1$ vorzugsweise für eine $C_4$-$C_{10}$-Alkylgruppe steht und $R_2$ und $R_3$ vorzugsweise für ein Wasserstoffatom oder eine Methylgruppe stehen,
- x und y, die gleich oder verschieden sein können, für eine ganze Zahl zwischen 1 und 100 stehen,
- z für eine ganze Zahl zwischen 0 und 100 steht,
- n für eine ganze Zahl zwischen 1 und 1000 steht.

**16.** Kit nach einem der Ansprüche 12 bis 15, wobei das Polysaccharid bzw. die Polysaccharide und das Polysaccharid-Derivat bzw. die Polysaccharid-Derivate aus Nitrocellulose und Ethern und Estern von Polysacchariden, insbesondere $C_2$-$C_4$-Ethern und -Estern von Polysacchariden, insbesondere aus Celluloseacetobutyraten, Celluloseacetopropionaten, Ethylcellulosen, Ethylguarverbindungen und Mischungen davon, weiter bevorzugt aus Nitrocellulose, ausgewählt ist bzw. sind.

**17.** Kit nach einem der Ansprüche 12 bis 16, wobei das filmbildende Polymer bzw. die filmbildenden Polymere in der ersten Zusammensetzung in einem Gesamtgehalt größer oder gleich 20 Gew.-%, bezogen auf das Gesamtgewicht der Feststoffe der ersten Zusammensetzung, bevorzugt von 25 bis 40 Gew.-%, bezogen auf das Gewicht der gesamten Feststoffe der ersten Zusammensetzung, vorliegt bzw. vorliegen.

**18.** Kit nach einem der Ansprüche 12 bis 17, wobei das filmbildende Polymer bzw. die filmbildenden Polymere in der zweiten Zusammensetzung in einem Gesamtgehalt größer oder gleich 0,05 Gew.-%, bezogen auf das Gesamtgewicht der Feststoffe der zweiten Zusammensetzung, insbesondere im Bereich von 0,1 bis 10 Gew.-%, bevorzugt von 0,2 bis 5 Gew.-%, bezogen auf das Gewicht der gesamten Feststoffe der zweiten Zusammensetzung, vorliegt

bzw. vorliegen.

**19.** Kit nach einem der vorhergehenden Ansprüche, wobei die erste Zusammensetzung mindestens ein flüchtiges Lösungsmittel, bevorzugt mindestens ein polares flüchtiges Lösungsmittel, das vorteilhafterweise aus der Gruppe bestehend aus $C_3$-$C_6$-Estern und -Ketonen und Mischungen davon ausgewählt ist, umfasst.

**20.** Kit nach Anspruch 19, wobei das flüchtige Lösungsmittel bzw. die flüchtigen Lösungsmittel in der ersten Zusammensetzung in einem Gesamtgehalt größer oder gleich 30 Gew.-%, bezogen auf das Gesamtgewicht der Zusammensetzung, insbesondere im Bereich von 50 % bis 70 %, bezogen auf das Gesamtgewicht der ersten Zusammensetzung, vorliegt bzw. vorliegen.

**21.** Kit nach einem der vorhergehenden Ansprüche, wobei die erste Zusammensetzung und die zweite Zusammensetzung mindestens einen Photoinitiator umfassen, wobei der Photoinitiator vorzugsweise aus der Gruppe bestehend aus $\alpha$-Hydroxyketonen, $\alpha$-Aminoketonen, aromatischen Ketonen, vorzugsweise in Kombination mit einem Wasserstoffdonor, aromatischen $\alpha$-Diketonen und Acylphosphinoxiden und Mischungen davon, vorteilhafterweise aus der Gruppe bestehend aus Acylphosphinoxiden, ausgewählt ist.

**22.** Kit nach einem der vorhergehenden Ansprüche, wobei die erste Zusammensetzung mindestens ein (ALK)acrylat-Monomer, wobei ALK für eine $C_1$-$C_6$-Alkylgruppe, bevorzugt eine $C_1$-$C_2$-Alkylgruppe, weiter bevorzugt eine $C_1$-Alkylgruppe, wie $CH_3$, steht, bevorzugt (Meth)acrylat-Monomer, weiter bevorzugt Tetrahydrofurfurylmethacrylat, umfasst.

**23.** Kit nach einem der vorhergehenden Ansprüche, wobei das (ALK)acrylat-Monomer bzw. die (ALK)acrylatMonomere, wobei ALK für eine $C_1$-$C_6$-Alkylgruppe, bevorzugt eine $C_1$-$C_2$-Alkylgruppe, weiter bevorzugt eine $C_1$-Alkylgruppe, wie $CH_3$, steht, in der ersten Zusammensetzung in einem Gesamtgehalt größer oder gleich 0,1 Gew.-%, bezogen auf das Gesamtgewicht der Feststoffe der ersten Zusammensetzung, insbesondere im Bereich von 0,2 bis 10 Gew.-%, vorzugsweise von 0,5 bis 5 Gew.-%, bezogen auf das Gewicht der gesamten Feststoffe der ersten Zusammensetzung, vorliegt bzw. vorliegen.

**24.** Kit nach einem der vorhergehenden Ansprüche, wobei das (ALK)acrylat-Monomer bzw. die (ALK)acrylatMonomere, wobei ALK für eine $C_1$-$C_6$-Alkylgruppe, bevorzugt eine $C_1$-$C_2$-Alkylgruppe, weiter bevorzugt eine $C_1$-Alkylgruppe, wie $CH_3$, steht, in der zweiten Zusammensetzung in einem Gesamtgehalt größer oder gleich 2 Gew.-%, bezogen auf das Gesamtgewicht der Feststoffe der zweiten Zusammensetzung, insbesondere im Bereich von 5 bis 40 Gew.-%, vorzugsweise von 10 bis 30 Gew.-%, bezogen auf das Gewicht der gesamten Feststoffe der zweiten Zusammensetzung, vorliegt bzw. vorliegen.

**25.** Verfahren zum Beschichten der Nägel und/oder künstlichen Nägel, insbesondere zum Zurechtmachen und/oder Pflegen der Nägel und/oder künstlichen Nägel, das mindestens die folgenden Schritte umfasst:

A) Aufbringen einer ersten photovernetzbaren Zusammensetzung des Kits nach einem der Ansprüche 1 bis 23 auf einen Nagel oder einen künstlichen Nagel, wodurch eine Beschichtung, die aus mindestens einer Schicht der ersten photovernetzbaren Zusammensetzung besteht, abgeschieden wird,
B) Einwirkenlassen von UV- oder sichtbarer Lichtstrahlung auf den am Ende von Schritt A) erhaltenen beschichteten Nagel oder künstlichen Nagel,
C) Aufbringen einer zweiten Zusammensetzung des Kits nach einem der Ansprüche 1 bis 23 auf die aus Schritt A) und B) resultierende erste Beschichtung, wodurch eine zweite Beschichtung, die aus mindestens einer Schicht der zweiten Zusammensetzung besteht, abgeschieden wird,
D) Einwirkenlassen von UV- oder sichtbarer Lichtstrahlung auf den am Ende von Schritt C) erhaltenen beschichteten Nagel oder künstlichen Nagel.

**26.** Verfahren zum Beschichten der Nägel und/oder künstlichen Nägel, insbesondere zum Zurechtmachen und/oder Pflegen der Nägel und/oder künstlichen Nägel, das mindestens die folgenden Schritte umfasst:

A) Aufbringen einer ersten photovernetzbaren Zusammensetzung des Kits nach einem der Ansprüche 1 bis 23 auf einen Nagel oder einen künstlichen Nagel, wodurch eine Beschichtung, die aus mindestens einer Schicht der ersten photovernetzbaren Zusammensetzung besteht, abgeschieden wird,
B) Einwirkenlassen von UV- oder sichtbarer Lichtstrahlung auf den am Ende von Schritt A) erhaltenen beschichteten Nagel oder künstlichen Nagel,

C) Aufbringen einer zweiten Zusammensetzung des Kits nach einem der Ansprüche 1 bis 23 auf die aus Schritt A) und B) resultierende erste Beschichtung, wodurch eine zweite Beschichtung, die aus mindestens einer Schicht der zweiten Zusammensetzung besteht, abgeschieden wird,
D) Einwirkenlassen von UV- oder sichtbarer Lichtstrahlung auf den am Ende von Schritt C) erhaltenen beschichteten Nagel oder künstlichen Nagel,
E) Aufbringen einer dritten Zusammensetzung, die von der ersten Zusammensetzung und von der zweiten Zusammensetzung verschieden ist, auf die aus Schritt C) und D) resultierende zweite Beschichtung, wodurch eine dritte Beschichtung, die aus mindestens einer Schicht der dritten Zusammensetzung besteht, abgeschieden wird,
F) Einwirkenlassen von UV- oder sichtbarer Lichtstrahlung auf den am Ende von Schritt E) erhaltenen beschichteten Nagel oder künstlichen Nagel.

**27.** Verfahren zum Beschichten der Nägel und/oder künstlichen Nägel nach Anspruch 26, bei dem dann, wenn eine dritte Beschichtung einer dritten Zusammensetzung aufgebracht wird, die als zweite Beschichtung aufgebrachte zweite Zusammensetzung mindestens ein Farbmittel umfasst.

**28.** Verfahren zum Beschichten der Nägel und/oder künstlichen Nägel nach Anspruch 26 oder 27, bei dem die Schritte C) und D) insgesamt zweimal wiederholt werden, wobei die zweite Zusammensetzung ein erstes Mal aufgebracht und UV- oder sichtbarer Lichtstrahlung ausgesetzt wird, dann ein zweites Mal aufgebracht und UV- oder sichtbarer Lichtstrahlung ausgesetzt wird, wobei die Schritte A) und B), wie E) und F), vorzugsweise jeweils nur einmal durchgeführt werden.

**Revendications**

**1.** Kit destiné au revêtement d'un ongle ou d'un faux ongle, et plus particulièrement destiné au maquillage d'un ongle ou d'un faux ongle, comprenant :

- une première composition photo-réticulable comprenant, dans un milieu physiologiquement acceptable, au moins un composé photo-réticulable a) comprenant au moins deux fonctions (ALK)acrylates, où ALK représente un groupement $C_1$-$C_6$-, préférablement $C_1$-$C_2$-, plus préférablement Ci-alkyle, tel que $CH_3$, et au moins une fonction acide carboxylique,
- une deuxième composition photo-réticulable comprenant dans un milieu physiologiquement acceptable :

    • au moins un composé photo-réticulable b) comprenant au moins un composé de poly(ALK)acrylate de (poly)uréthane, où ALK représente un groupement $C_1$-$C_6$-, préférablement $C_1$-$C_2$-, plus préférablement Ci-alkyle, tel que $CH_3$, préférablement un composé de di(méth)acrylate de polyuréthane, plus préférentiellement un composé de diméthacrylate de polyuréthane, le ou les composés photoréticulables b) comprenant un motif (poly)oxyalkylène, en particulier comprenant un motif (poly)oxyéthylène, comprenant de préférence de 1 à 100 unités oxyalkylène, de préférence de 5 à 50 unités oxyalkylène, et préférentiellement environ 8 à 10 unités oxyalkylène, de préférence identique à celui contenu dans la première composition,
    • au moins un composé photo-réticulable c) comprenant au moins deux motifs carbamates obtenus par réaction avec au moins un diisocyanate de type diisocyanate d'isophorone, le ou les composés c) correspondant à la formule (XIII) ci-après :

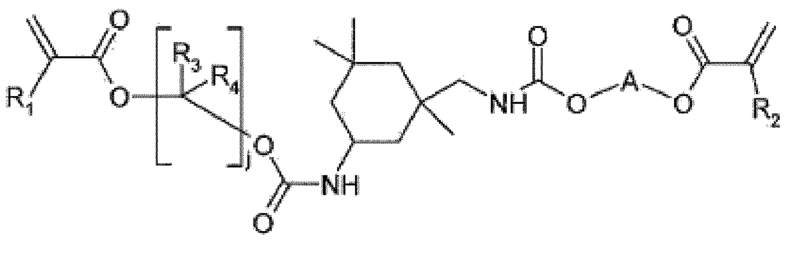

Formule (XIII)

formule (XIII) dans laquelle :

- $R_1$, $R_2$, $R_3$ et $R_4$, qui peuvent être qui peuvent être identiques ou différents, représentent un atome d'hydrogène ou une chaîne $C_1$-$C_{10}$-alkyle, préférablement un atome d'hydrogène ou un groupement méthyle,
- j est compris entre 1 et 10, préférablement est égal à 2,
- A représente un groupement $C_1$-$C_{10}$-alkyle, ou un polyuréthane comprenant de 2 à 20 unités carbamate,

• au moins un monomère de (ALK)acrylate, où ALK représente un groupement $C_1$-$C_6$-, préférablement $C_1$-$C_2$-, plus préférablement Ci-alkyle, tel que $CH_3$, de préférence un monomère de (méth)acrylate, plus préférentiellement le méthacrylate de tétrahydrofurfuryle.

**2.** Kit selon la revendication 1, dans lequel le ou les composés a) correspondent à la formule (I) ci-après :

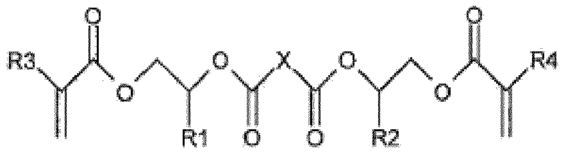

(I)

formule (I) dans laquelle :

- R1 et R2, qui peuvent être identiques ou différents, représentent un atome d'hydrogène, ou un groupement méthyle, ou un groupement de formule (II) ci-après :

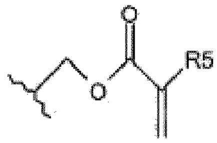

(II)

R5 représentant un atome d'hydrogène ou un groupement méthyle ;
- R3 et R4, qui peuvent être identiques ou différents, représentent un atome d'hydrogène ou un groupement méthyle ;
- X représente un radical correspondant à l'une des formules (III), (IV), (V), (VI), (VII), (VIII), (IX), ou (X) ci-après :

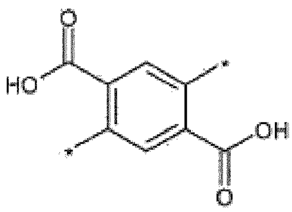

(III)

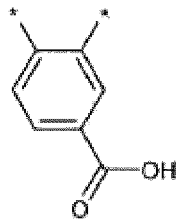

(IV)

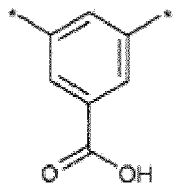

(V)

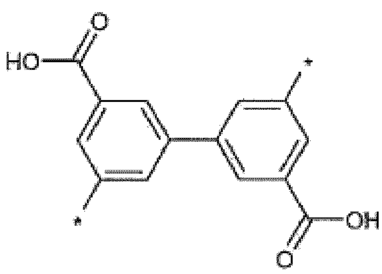

(VI)

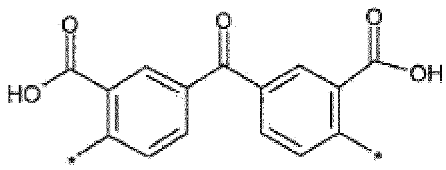

(VII)

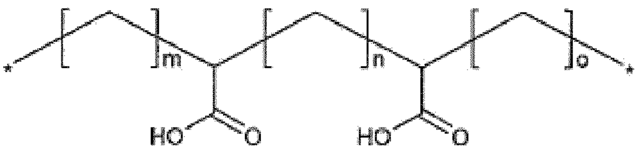

(VIII)

m, n, et o, qui peuvent être identiques ou différents, représentant un nombre entier compris entre 0 et 10 ;

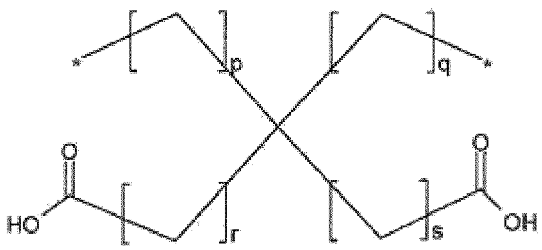

(IX)

p, q, r, et s, qui peuvent être identiques ou différents, représentant un nombre entier compris entre 0 et 10 ;

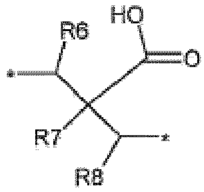

(X)

R6, R7 et R8, qui peuvent être identiques ou différents, représentant un atome d'hydrogène ou un groupement hydroxyle.

**3.** Kit selon la revendication 2, dans lequel le ou les composés a) correspondent à la formule (I-1) ci-après :

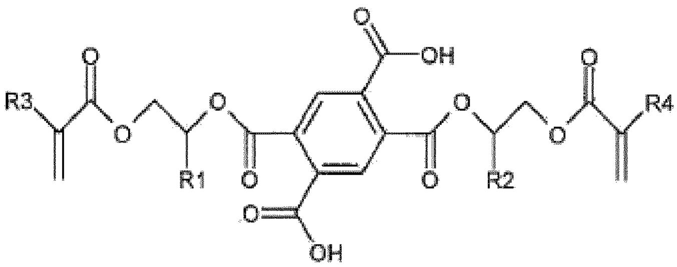

(I-1)

formule (I-1) dans laquelle R1, R2, R3 et R4 sont tels que définis selon la revendication 2.

**4.** Kit selon la revendication 3, dans lequel R3 et R4 de la formule (I-1) sont des groupements méthyle.

**5.** Kit selon la revendication 3 ou 4, dans lequel R1 et R2 de la formule (I-1) représentent des atomes d'hydrogène.

**6.** Kit selon l'une quelconque des revendications 2 à 5, dans lequel le ou les composés a), correspondant à la formule (I), sont présents dans la première composition selon une teneur supérieure ou égale à 10% en poids, par rapport au poids des matières solides totales de la première composition, en particulier allant de 10% à 25% en poids, de préférence de 15% à 20% en poids, par rapport au poids des matières solides totales de la première composition.

**7.** Kit selon l'une quelconque des revendications précédentes, dans lequel la première composition comprend au moins un composé photo-réticulable b) comprenant au moins un composé de poly(ALK)acrylate de (poly)uréthane, où ALK représente un groupement $C_1$-$C_6$-, préférablement $C_1$-$C_2$-, plus préférablement Ci-alkyle, tel que $CH_3$, de préférence un composé de di(méth)acrylate de polyuréthane, plus préférentiellement un composé de diméthacrylate de polyuréthane, le ou les composés photoréticulables b) comprenant un motif (poly)oxyalkylène, en particulier

comprenant un motif (poly)oxyéthylène, comprenant de préférence de 1 à 100 unités oxyalkylène, de préférence de 5 à 50 unités oxyalkylène, et préférentiellement environ 8 à 10 unités oxyalkylène, de préférence identique à celui contenu dans la première composition.

**8.** Kit selon l'une quelconque des revendications précédentes, dans lequel le ou les composés b) correspondent à la formule (XI) ci-après :

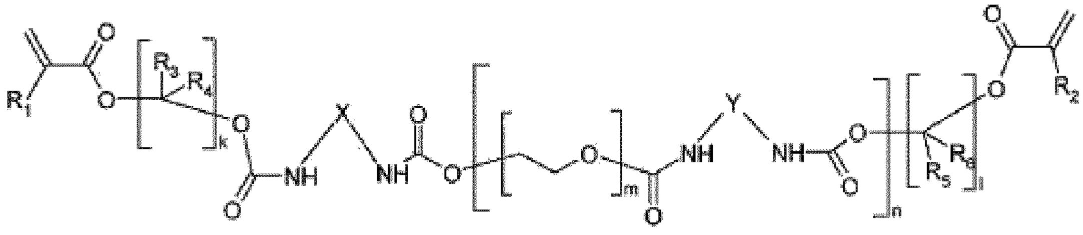

Formule (XI)

formule (XI) dans laquelle :

- $R_1$, $R_2$, $R_3$, $R_4$, $R_5$, et $R_6$, qui peuvent être identiques ou différents, représentent un atome d'hydrogène ou une chaine $C_1$-$C_6$-alkyle, de préférence un atome d'hydrogène ou un groupement méthyle,
- k et l, qui peuvent être identiques ou différents, sont compris entre 1 et 10, de préférence égaux à 2,
- m est compris entre 1 et 100, de préférence entre 5 et 50, de préférence entre environ 8 et 10,
- n est compris entre 1 et 10, de préférence égal à 1,
- X et Y, qui peuvent être identiques ou différents, représentent un groupement $C_1$-$C_{20}$-alkyle ou -cycloalkyle.

**9.** Kit selon la revendication 7 ou 8, dans lequel le ou les composés b) présents dans la première composition, en particulier correspondant à la formule (XI), sont présents selon une teneur supérieure ou égale à 20% en poids, par rapport au poids total des matières solides de la première composition, en particulier allant de 25% à 50% en poids, de préférence de 30% à 50% en poids, par rapport au poids des matières solides totales de la première composition.

**10.** Kit selon l'une quelconque des revendications précédentes, dans lequel le ou les composés b) présents dans la deuxième composition, en particulier correspondant à la formule (XI), sont présents selon une teneur supérieure ou égale à 10% en poids, par rapport au poids total des matières solides de la deuxième composition, en particulier allant de 25% à 80% en poids, de préférence de 50% à 70% en poids, par rapport au poids des matières solides totales de la deuxième composition.

**11.** Kit selon l'une quelconque des revendications précédentes, dans lequel le ou les composés c), correspondant à la formule (XIII), sont présents dans la deuxième composition selon une teneur supérieure ou égale à 5% en poids, par rapport au poids total des matières solides de la deuxième composition, en particulier allant de 10% à 80% en poids, de préférence de 15% à 70% en poids, par rapport au poids des matières solides totales de la deuxième composition.

**12.** Kit selon l'une quelconque des revendications précédentes, dans lequel la première composition et la deuxième composition comprennent au moins un polymère filmogène, de préférence choisi dans le groupe constitué par les poly(méth)acrylates, les polysaccharides et dérivés, et un mélange de ceux-ci, de préférence un mélange de ceux-ci.

**13.** Kit selon l'une quelconque des revendications précédentes, dans lequel la première composition comprend au moins un polymère filmogène choisi dans le groupe constitué par les poly(méth)acrylates, et de préférence un mélange de poly(méth)acrylate(s) et de polysaccharide(s) et dérivés.

**14.** Kit selon l'une quelconque des revendications précédentes, dans lequel la deuxième composition comprend au moins un polymère filmogène choisi dans le groupe constitué par les polysaccharides et dérivés.

**15.** Kit selon l'une quelconque des revendications 12, 13 ou 14, dans lequel le ou les polymères filmogènes comprennent au moins un poly(méth)acrylate correspondant à la formule (XII) ci-après :

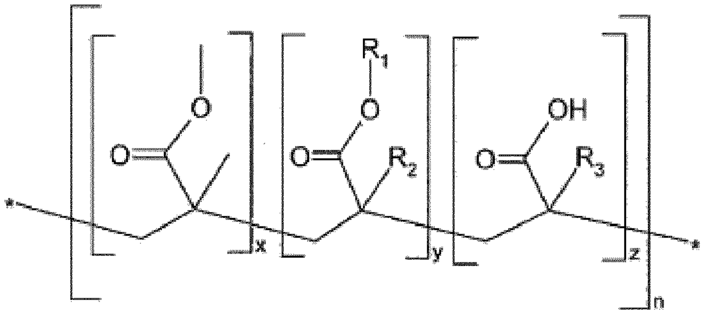

Formule (XII)

formule (XII) dans laquelle :

- $R_1$, $R_2$ et $R_3$, qui peuvent être identiques ou différents, représentent un atome d'hydrogène ou un groupement $C_1$-$C_{10}$-alkyle, $R_1$ représentant de préférence un groupement $C_4$-$C_{10}$-alkyle, et $R_2$ et $R_3$ représentant de préférence un atome d'hydrogène ou un groupement méthyle,
- x et y, qui peuvent être identiques ou différents, représentent un nombre entier compris entre 1 et 100,
- z représente un nombre entier compris entre 0 et 100,
- n représentant un nombre entier compris entre 1 et 1000.

**16.** Kit selon l'une quelconque des revendications 12 à 15, dans lequel le ou les polysaccharides et dérivés de polysaccharides sont choisis parmi la nitrocellulose et les éthers et esters de polysaccharides, notamment en $C_2$-$C_4$, notamment parmi les acétobutyrates de cellulose, les acétopropionates de cellulose, les éthylcelluloses, les éthyl guars, et des mélanges de ceux-ci, plus préférentiellement choisis parmi la nitrocellulose.

**17.** Kit selon l'une quelconque des revendications 12 à 16, dans lequel le ou les polymères filmogènes sont présents dans la première composition selon une teneur totale supérieure ou égale à 20% en poids, par rapport au poids total des matières solides de la première composition, de préférence de 25% à 40% en poids, par rapport au poids des matières solides totales de la première composition.

**18.** Kit selon l'une quelconque des revendications 12 à 17, dans lequel le ou les polymères filmogènes sont présents dans la deuxième composition selon une teneur totale supérieure ou égale à 0,05% en poids, par rapport au poids total des matières solides de la deuxième composition, en particulier allant de 0,1% à 10% en poids, de préférence de 0,2% à 5% en poids, par rapport au poids des matières solides totales de la deuxième composition.

**19.** Kit selon l'une quelconque des revendications précédentes, dans lequel la première composition comprend au moins un solvant volatil, de préférence au moins un solvant volatil polaire choisi de manière avantageuse dans le groupe constitué par les esters et cétones en $C_3$-$C_6$ et des mélanges de ceux-ci.

**20.** Kit selon la revendication 19, dans lequel le ou les solvants volatils sont présents dans la première composition selon une teneur totale supérieure ou égale à 30% en poids, par rapport au poids total de la composition, en particulier allant de 50% à 70% par rapport au poids total de la première composition.

**21.** Kit selon l'une quelconque des revendications précédentes, dans lequel la première composition et la deuxième composition comprennent au moins un photo-initiateur, le photo-initiateur étant choisi de préférence dans le groupe constitué par les $\alpha$-hydroxycétones, les $\alpha$-aminocétones, les cétones aromatiques associées de préférence à un composé donneur d'hydrogène, les $\alpha$-dicétones aromatiques et les oxydes d'acylphosphine, et des mélanges de ceux-ci, de manière avantageuse dans le groupe constitué par les oxydes d'acylphosphine.

**22.** Kit selon l'une quelconque des revendications précédentes, dans lequel la première composition comprend au moins un monomère de (ALK)acrylate, où ALK représente un groupement $C_1$-$C_6$-, préférablement $C_1$-$C_2$-, plus préférablement Ci-alkyle, tel que $CH_3$, de préférence un monomère de (méth)acrylate, plus préférentiellement le méthacrylate de tétrahydrofurfuryle.

**23.** Kit selon la revendication précédente, dans lequel le ou les monomères de (ALK)acrylate, où ALK représente un groupement $C_1$-$C_6$-, préférablement $C_1$-$C_2$-, plus préférablement Ci-alkyle, tel que $CH_3$, sont présents dans la

première composition selon une teneur totale supérieure ou égale à 0,1% en poids, par rapport au poids total des matières solides de la première composition, en particulier allant de 0,2% à 10% en poids, de préférence de 0,5% à 5% en poids, par rapport au poids des matières solides totales de la première composition.

**24.** Kit selon l'une quelconque des revendications précédentes, dans lequel le ou les monomères de (ALK)acrylate, où ALK représente un groupement $C_1$-$C_6$-, préférablement $C_1$-$C_2$-, plus préférablement Ci-alkyle, tel que $CH_3$, sont présents dans la deuxième composition selon une teneur totale supérieure ou égale à 2% en poids, par rapport au poids total des matières solides de la deuxième composition, en particulier allant de 5% à 40% en poids, de préférence de 10% à 30% en poids, par rapport au poids des matières solides totales de la deuxième composition.

**25.** Méthode de revêtement des ongles et/ou des faux ongles, en particulier de maquillage et/ou de soin des ongles et/ou des faux ongles, comprenant au moins les étapes suivantes :

A) l'application, sur un ongle ou un faux ongle, d'une première composition photo-réticulable du kit selon l'une quelconque des revendications 1 à 23, où on dépose un revêtement constitué d'au moins une couche de ladite première composition photo-réticulable,
B) l'exposition de l'ongle ou du faux ongle revêtu obtenu à l'issue de l'étape A) à un rayonnement lumineux, UV ou visible,
C) l'application, sur le premier revêtement issu de l'étape A) et B), d'une deuxième composition du kit selon l'une quelconque des revendications 1 à 23, où on dépose un deuxième revêtement constitué d'au moins une couche de ladite deuxième composition,
D) l'exposition de l'ongle ou du faux ongle revêtu obtenu à l'issue de l'étape C) à un rayonnement lumineux, UV ou visible.

**26.** Méthode de revêtement des ongles et/ou des faux ongles, en particulier de maquillage et/ou de soin des ongles et/ou des faux ongles, comprenant au moins les étapes suivantes :

A) l'application, sur un ongle ou un faux ongle, d'une première composition photo-réticulable du kit selon l'une quelconque des revendications 1 à 23, où on dépose un revêtement constitué d'au moins une couche de ladite première composition photo-réticulable,
B) l'exposition de l'ongle ou du faux ongle revêtu obtenu à l'issue de l'étape A) à un rayonnement lumineux, UV ou visible,
C) l'application, sur le premier revêtement issu de l'étape A) et B), d'une deuxième composition du kit selon l'une quelconque des revendications 1 à 23, où on dépose un deuxième revêtement constitué d'au moins une couche de ladite deuxième composition,
D) l'exposition de l'ongle ou du faux ongle revêtu obtenu à l'issue de l'étape C) à un rayonnement lumineux, UV ou visible,
E) l'application, sur le deuxième revêtement issu de l'étape C) et D), d'une troisième composition, distincte de la première composition et de la deuxième composition, où on dépose un troisième revêtement constitué d'au moins une couche de ladite troisième composition,
F) l'exposition de l'ongle ou du faux ongle revêtu obtenu à l'issue de l'étape E) à un rayonnement lumineux, UV ou visible.

**27.** Méthode de revêtement des ongles et/ou des faux ongles selon la revendication 26, dans laquelle, lorsqu'un troisième revêtement d'une troisième composition est appliqué, la deuxième composition appliquée en tant que deuxième revêtement comprend au moins un agent de coloration.

**28.** Méthode de revêtement des ongles et/ou des faux ongles selon la revendication 26 ou 27, dans laquelle les étapes C) et D) sont répétées au total deux fois, la deuxième composition étant appliquée une première fois et exposée à un rayonnement lumineux, UV ou visible, puis étant appliquée une deuxième fois et exposée à un rayonnement lumineux, UV ou visible, les étapes A) et B), de même que E) et F), n'étant de préférence mises en oeuvre respectivement qu'une seule fois.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CA 1306954
- US 5456905 A
- US 7375144 B
- FR 2823105
- US 20110081306 A
- US 20110082228 A
- US 20110274633 A
- US 20120083547 A
- DE 102011102661


### Non-patent literature cited in the description


- **C.M. HANSEN.** The three dimensional solubility parameters. *J. Paint Technol.,* 1967, vol. 39 (105
- **G. LI BASSI.** Les photoinitiateurs dans la reticulation des revetements'' [''Photoinitiators in the crosslinking of coatings. *Double Liaison - Chimie des Peintures,* November 1985, 34-41
- **HENRI STRUB.** Applications industrielles de la polymerisation photoinduite'' [''Industrial applications of photoinduced polymerization. *L'Actualite Chimique,* February 2000, 5-13
- **MARC, J.M. ABADIE.** Photopolymères: considerations théoriques et reaction de prise'' [''Photopolymers: theoretical considerations and setting reaction. *Double Liaison - Chimie des Peintures,* 1992, 28-34
- **HENRI STRUB.** Applications industrielles de la polymériation photoinduite'' [''Industrial applications of photoinduced polymerization. *L'Actualité Chimique,* February 2000, 5-13